(19) **Europäisches Patentamt / European Patent Office / Office européen des brevets**

(11) **EP 1 962 212 A1**

(12) **EUROPEAN PATENT APPLICATION**

(43) Date of publication:
27.08.2008 Bulletin 2008/35

(51) Int Cl.:
*G06F 19/00* (2006.01)     *A01H 1/04* (2006.01)

(21) Application number: 07290060.8

(22) Date of filing: 17.01.2007

| | |
|---|---|
| (84) Designated Contracting States:<br>AT BE BG CH CY CZ DE DK EE ES FI FR GB GR HU IE IS IT LI LT LU LV MC NL PL PT RO SE SI SK TR<br>Designated Extension States:<br>AL BA HR MK RS<br><br>(71) Applicant: **Syngenta Participations AG**<br>4058 Basel (CH)<br><br>(72) Inventors:<br>• **Ragot Michel**<br>31000 Toulouse (FR)<br>• **Gay Gilles**<br>31000 Toulouse (FR) | • **Fisch Roland**<br>4112 Flüh (CH)<br>• **Willé David**<br>Bishop's Stortford CM23 2HE (GB)<br>• **Lespinasse Denis**<br>31340 La Magdelaine sur Tarn (FR)<br>• **Lhermine Michel**<br>31500 Toulouse (FR)<br>• **Argillier Odile**<br>78120 Rambouillet (FR)<br><br>(74) Representative: **Vossius & Partner**<br>Siebertstrasse 3<br>81675 München (DE) |

(54) **Process for selecting individuals and designing a breeding program**

(57)    The presently disclosed subject matter provides methods for improving the efficacy of a plant breeding program aimed at altering phenotypic traits for which associations with genetic markers can be established. Genome-wide genetic values of individuals are computed based on the individuals' marker genotypes and the associations established between genetic markers and phenotypic traits. Individuals and breeding schemes are then selected based both on the individuals' genome-wide genetic value and on the distributions of these genetic values for the potential progenies derived through the breeding schemes under evaluation. The presently disclosed subject matter also provides systems and computer program products for performing the disclosed methods as well as plants selected, provided, or produced by any of the methods herein and transgenic plants created by any of the methods herein.

FIGURE 2

**Description**

TECHNICAL FIELD

The presently disclosed subject matter relates to methods for improving the efficacy of a plant breeding program. In some embodiments, the plant breeding program is aimed at altering phenotypic traits for which associations with genetic markers can be established. Genetic values of individuals can be computed based on the individuals' marker genotypes and the associations established between genetic markers and phenotypic traits. Individuals and mating schemes can then be selected based both on the individuals' genome-wide genetic value and on the distributions of these genetic values for the potential progenies derived through the mating schemes under evaluation. The presently disclosed subject matter also relates to systems and computer program products for performing the disclosed methods as well as plants selected, provided, or produced by, and transgenic plants created by, the disclosed methods.

BACKGROUND ART

Selective breeding has been employed for centuries to improve, or attempt to improve, phenotypic traits of agronomic and economic interest in plants, such as yield, percentage of grain oil, etc. Generally speaking, selective breeding involves the selection of individuals to serve as parents of the next generation on the basis of one or more phenotypic traits of interest. However, such phenotypic selection is frequently complicated by non-genetic factors that can impact the phenotype(s) of interest. Non-genetic factors that can have such effects include, but are not limited to environmental influences such as soil type and quality, rainfall, temperature range, and others.

Another significant problem with breeding strategies that rely on phenotypic selection is that most phenotypic traits of interest are controlled by more than one genetic locus, each of which typically influences the given trait to a greater or lesser degree. For example, U.S. Patent No. 6,399,855 to Beavis suggests that the vast majority of economically important phenotypic traits in domesticated plants are so-called quantitative traits. Generally, the term "quantitative trait" has been used to describe a phenotype that exhibits continuous variability in expression and is the net result of multiple genetic loci presumably interacting with each other and/or with the environment. The term "complex trait" has also been broadly used to describe any trait that does not exhibit classic Mendelian inheritance, which generally is attributable to a single genetic locus (Lander & Schork, 1994).

One of the consequences of multi-factorial inheritance patterns is that it can be very difficult to map loci that contribute to the expression of such traits. However, the development of sets of polymorphic genetic markers (e.g., RFLPs, SNPs, SSRs, etc.) that span the genome has made it possible to investigate what Edwards *et al.* referred to as "quantitative trait loci" (QTL or QTLs; Edwards *et al.,* 1987), as well as their numbers, magnitudes, and distributions. QTLs include genes that control, to some degree, qualitative and quantitative phenotypic traits that can be discrete or continuously distributed within a family of individuals as well as within a population of families of individuals.

Various experimental approaches have been developed to identify and analyze QTLs (see *e.g.,* U.S. Patent Nos. 5,385,835; 5,492,547; and 5,981,832). One such approach involves crossing two inbred lines to produce $F_1$ single cross hybrid progeny, selfing the $F_1$ hybrid progeny to produce segregating $F_2$ progeny, genotyping multiple marker loci, and evaluating one to several quantitative phenotypic traits among the segregating progeny. The QTLs are then identified on the basis of significant statistical associations between the genotypic values and the phenotypic variability among the segregating progeny. The parental lines of the $F_1$ generation have known linkage phases, all of the segregating loci in the progeny are informative, and linkage disequilibrium between the marker loci and the genetic loci affecting the phenotypic traits is maximized.

However, considerable resources must be devoted to determining the phenotypic performance of large numbers of hybrid and/or inbred progeny. Because the progeny from only two parents are studied, this approach can only detect the trait loci (e.g., the QTLs) for which the two parents are polymorphic. This set of trait loci might only represent a fraction of the loci segregating in breeding populations of interest (e.g., breeding populations of maize, sorghum, soybean, canola, etc.). In general, these progeny show variation for only one or a small number of the phenotypic traits that are of interest in applied breeding programs. This means that separate populations might need to be developed, scored for marker loci, and grown in replicated field experiments and scored for the phenotypic traits of interest. Additionally, methods used to detect QTLs can produce biased estimates of the QTLs that are identified *(see e.g.,* Beavis, 1994). Additional imprecision can be introduced in extrapolating the identification of QTLs to the progeny of genetically different parents within a breeding population. Furthermore, many if not all traits are affected by environmental factors, which can also introduce imprecision.

Thus, there is a long-standing and continuing need for new methods for optimizing breeding strategies for producing progeny with desirable genotypes. This and other needs are addressed by the presently disclosed subject matter.

SUMMARY

This Summary lists several embodiments of the presently disclosed subject matter, and in many cases lists variations and permutations of these embodiments. This Summary is merely exemplary of the numerous and varied embodiments. Mention of one or more representative features of a given embodiment is likewise exemplary. Such an embodiment can typically exist with or without the feature(s) mentioned; likewise, those features can be applied to other embodiments of the presently disclosed subject matter, whether listed in this Summary or not. To avoid excessive repetition, this Summary does not list or suggest all possible combinations of such features.

The presently disclosed subject matter provides methods for calculating a distribution of a probability or frequency of occurrence of one or more potential genotypes. In some embodiments, the presently disclosed methods comprise (a) providing a first breeding partner and a second breeding partner, wherein (i) the genotype of each of the first breeding partner and the second breeding partner is known or is predictable with respect to one or more genetic markers, each of which is linked to a genetic locus; and (ii) a genetic distance between each genetic marker and the genetic locus to which it is linked is known or can be assigned; (b) calculating, simulating, or combinations of calculating and simulating a breeding of the first breeding partner and the second breeding partner to generate a subsequent generation, each member of the subsequent generation comprising a genotype; and (c) calculating a distribution of a probability or a frequency of occurrence for one or more of the genotypes of one or more members of the subsequent generation.

The presently disclosed subject matter also provides methods for calculating a genetic value distribution. In some embodiments, the presently disclosed methods comprise (a) providing a first breeding partner and a second breeding partner, wherein (i) the genotype of each of the first breeding partner and the second breeding partner is known or is predictable with respect to one or more genetic markers linked to one or more genetic loci; (ii) a genetic distance between each genetic marker and the genetic locus to which it is linked is known or can be assigned; and (iii) each genotype is associated with a genetic value; (b) calculating, simulating, or combinations of calculating and simulating a breeding of the first breeding partner and the second breeding partner to generate a subsequent generation, each member of the subsequent generation comprising a genotype; and (c) calculating a genetic value distribution for one or more of the genotypes.

The presently disclosed subject matter also provides methods for choosing a breeding pair for producing a progeny having a desired genotype. In some embodiments, the presently disclosed methods comprise (a) providing a first breeding partner and a second breeding partner, wherein (i) the genotype of each of the first breeding partner and the second breeding partner is known or is predictable with respect to one or more genetic markers, each of which is linked to a genetic locus; and (ii) a genetic distance between each genetic marker and the genetic locus to which it is linked is known or can be assigned; (b) calculating, simulating, or combinations of calculating and simulating a breeding of the first breeding partner and the second breeding partner to generate a subsequent generation, each member of the subsequent generation comprising a genotype; (c) calculating a distribution of a probability or a frequency of occurrence for one or more of the genotypes of one or more members of the subsequent generation; (d) repeating steps (a) through (c) with a different first, different second, or both different first and different second potential breeding partners; (e) comparing the probability or frequency distributions calculated in one or more iterations of step (c) to each other; and (f) choosing a breeding pair based on the comparing step.

In some embodiments, the presently disclosed methods for choosing a breeding pair for producing a progeny having a desired genotype comprise (a) providing a first breeding partner and a second breeding partner, wherein (i) the genotype of each of the first breeding partner and the second breeding partner is known or is predictable with respect to one or more genetic markers linked to one or more genetic loci; (ii) a genetic distance between each genetic marker and the genetic locus to which it is linked is known or can be assigned; and (iii) each genotype is associated with a genetic value; (b) calculating, simulating, or combinations of calculating and simulating a breeding of the first breeding partner and the second breeding partner to generate a subsequent generation, each member of the subsequent generation comprising a genotype; (c) calculating a distribution of genetic values associated with one or more of the genotypes of one or more members of the subsequent generation; (d) repeating steps (a) through (c) with a different first, different second, or both different first and different second potential breeding partners; (e) comparing the genetic value distributions calculated in one or more iterations of step (c) to each other; and (f) choosing a breeding pair based on the comparing step.

The presently disclosed subject matter also provides methods for generating a progeny individual having a desired genotype. In some embodiments, the presently disclosed methods comprise (a) providing a first breeding partner and a second breeding partner, wherein (i) the genotype of each of the first breeding partner and the second breeding partner is known or is predictable with respect to one or more genetic markers, each of which is linked to a genetic locus; and (ii) a genetic distance between each genetic marker and the genetic locus to which it is linked is known or can be assigned; (b) calculating, simulating, or combinations of calculating and simulating a breeding of the first breeding partner and the second breeding partner to generate a subsequent generation, each member of the subsequent generation comprising a genotype; (c) calculating a distribution of a probability or a frequency of occurrence for one or more of the genotypes of one or more members of the subsequent generation; (d) repeating steps (a) through (c) with a different first, different second, or both different first and different second potential breeding partners; (e) comparing the probability or frequency distributions calculated in one or more iterations of step (c) to each other; (f) choosing a breeding pair based on the comparing step; and (g) breeding the breeding pair in accordance with the calculating, simulating, or combinations of calculating and simulating as set forth in step (b) to generate a progeny individual having a desired genotype.

In some embodiments, the presently disclosed methods for generating a progeny individual having a desired genotype comprises (a) providing a first breeding partner and a second breeding partner, wherein (i) the genotype of each of the first breeding partner and the second breeding partner is known or is predictable with respect to one or more genetic markers linked to one or more genetic loci; (ii) a genetic distance between each genetic marker and the genetic locus to which it is linked is known or can be assigned; and (iii) each genotype is associated with a genetic value; (b) calculating, simulating, or combinations of calculating and simulating a breeding of the first breeding partner and the second breeding partner to generate a subsequent generation, each member of the subsequent generation comprising a genotype; (c) calculating a distribution of genetic values associated with one or more of the genotypes of one or more members of the subsequent generation; (d) repeating steps (a) through (c) with a different first, different second, or both different first and different second potential breeding partners; (e) comparing the genetic value distributions calculated in one or more iterations of step (c) to each other; (f) choosing a breeding pair based on the comparing step; and (g) breeding the breeding pair in accordance with the calculating, simulating, or combinations of calculating and simulating as set forth in step (b) to generate a progeny individual having a desired genotype.

In some embodiments, the presently disclosed methods further comprise generating one or more further generation progeny, wherein each further generation progeny is generated by one or more rounds of calculating, simulating, or combinations of calculating and simulating a breeding of at least one member of the subsequent generation or a later generation with an individual selected from the group consisting of itself, a member of the immediately prior generation, another individual from the same generation, another individual from a previous generation, the first breeding partner, the second breeding partner, and doubled haploid derivatives thereof.

In some embodiments, the further generation progeny are generated by one or more successive generations of crossings, selfings, doubled haploid derivative generation, or combinations thereof of one or more individuals from a preceding generation. In some embodiments, the further generation progeny are generated by three successive generations of crossings, selfings, doubled haploid derivative generation, or combinations thereof of one or more individuals of a preceding generation. In some embodiments, the further generation progeny are generated by four successive generations of crossings, selfings, doubled haploid derivative generation, or combinations thereof of one or more individuals from a preceding generation. In some embodiments, the further generation is generated by at least two, three, or four successive generations of selfing of one or more members of a preceding generation.

In some embodiments of the presently disclosed methods, the one or more genetic markers are selected from the group consisting of a single nucleotide polymorphism (SNP), an indel (i.e., insertion/deletion), a simple sequence repeat (SSR), a restriction fragment length polymorphism (RFLP), a random amplified polymorphic DNA (RAPD), a cleaved amplified polymorphic sequence (CAPS) marker, a Diversity Arrays Technology (DArT) marker, an amplified fragment length polymorphism (AFLP), and combinations thereof. In some embodiments, the one or more genetic markers comprise between one and ten markers. In some embodiments, the one or more genetic markers comprise more than ten genetic markers.

In some embodiments of the presently disclosed methods, the calculating, simulating, or combinations of calculating and simulating a breeding includes calculating, simulating, or combinations of calculating and simulating an expected rate of recombination between at least one of the one or more genetic markers and a genetic locus associated with expression of a phenotypic trait.

In some embodiments of the presently disclosed methods, the phenotypic trait is selected from the group consisting of a qualitative trait and a quantitative trait.

In some embodiments, the one or more genetic markers are linked to one or more quantitative trait loci associated with expression of the phenotypic trait.

In some embodiments, the genetic locus associated with expression of the phenotypic trait encodes a gene product that is associated with expression of the phenotypic trait.

In some embodiments, the rate of recombination between the at least one of the one or more genetic markers and the genetic locus associated with expression of the phenotypic trait is zero.

In some embodiments of the presently disclosed methods, the breeding partners are the same individual.

In some embodiments of the presently disclosed methods, each calculated or simulated breeding comprises selfing an individual from the immediately prior generation.

In some embodiments of the presently disclosed methods, the breeding pair comprises a pool of male genotypes, a pool of female genotypes, or both a pool of male and a pool of female genotypes.

The presently disclosed subject matter also provides individuals generated by the presently disclosed methods. In some embodiments, an individual so generated is a plant. In some embodiments, the presently disclosed subject matter also provides cells, seed, and/or progeny from the plant generated by the presently disclosed methods.

Accordingly, it is an object of the presently disclosed subject matter to provide new methods for designing a breeding program. This and other objects are achieved in whole or in part by the presently disclosed subject matter.

An object of the presently disclosed subject matter having been stated hereinabove, other objects will be evident as the description proceeds and as best described hereinbelow.

BRIEF DESCRIPTION OF THE DRAWINGS

Figure 1 illustrates an exemplary general purpose computing platform **100** upon which the methods and systems of the presently disclosed subject matter can be implemented.

Figure 2 is a flowchart of a process **200** for implementing a method for calculating a distribution of a probability or a frequency of occurrence of one or more potential genotypes as disclosed herein.

Figure 3 is a flowchart of a process **300** for implementing a method for calculating a genetic value distribution as disclosed herein.

Figure 4 is a flowchart of a process **400** for implementing a method for choosing a breeding pair for producing a progeny having a desired genotype as disclosed herein.

Figure 5 is a flowchart of a process **500** for implementing a method for generating a progeny individual having a desired genotype as disclosed herein.

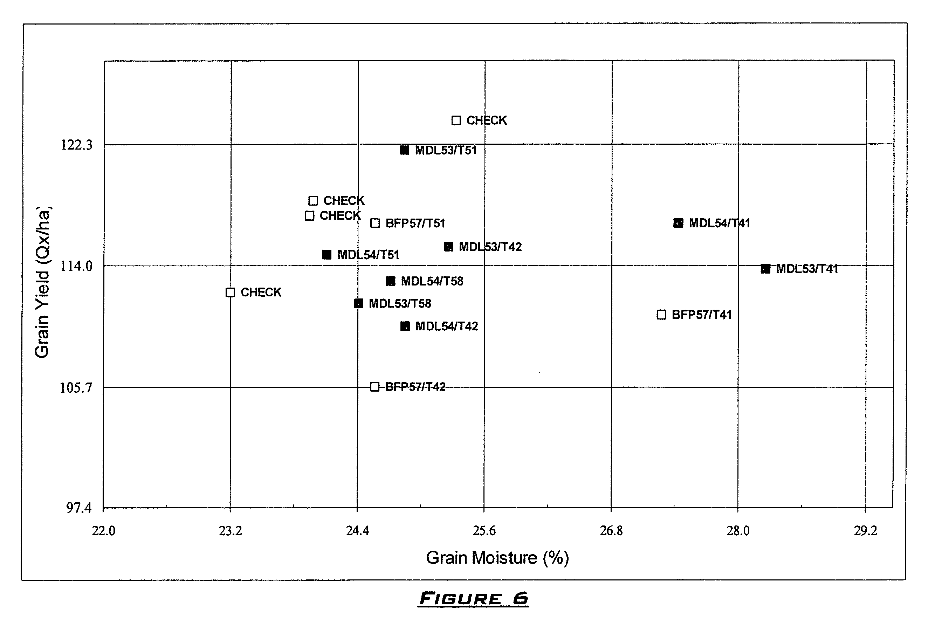

Figure 6 is a plot depicting agronomic performance of marker-based-selection-derived material, compared to reference material. Figure 6 shows grain yield (in quintals per hectare) and grain moisture at harvest of hybrids made from two marker-based-selection-derived lines, MDL53 and MDL54, crossed onto four testers, T41, T42, T51, and T58, and grown at five locations in Europe in 2006. The results shown are the averages over all five locations. The figure also shows performance of reference commercial hybrids (identified as "check") as well as performance of one parental line, BFP57, crossed onto T41, T42, and T51. Check hybrids are represented by white squares. Marker-based-selection-derived hybrids are represented by black squares. The hybrids that show high grain yield and low grain moisture at harvest are positioned in the upper left corner of Figure 6.

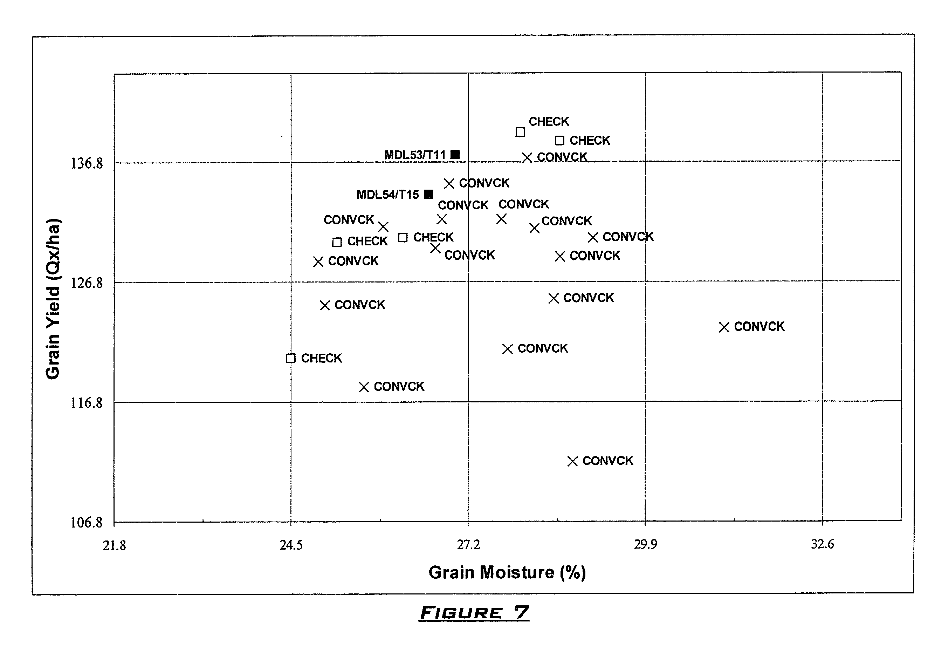

Figure 7 is a plot depicting agronomic performance of marker-based-selection-derived material, compared to reference material. Figure 7 shows grain yield (in quintals per hectare) and grain moisture at harvest of hybrids made from two marker-based-selection-derived lines, MDL53 and MDL54, crossed onto two testers, T11 and T15, and grown at four locations in Europe in 2006. The results shown are the averages over all four locations. The figure also shows performance of reference commercial hybrids (identified as "check") as well as performance of experimental hybrids derived through conventional breeding. Check hybrids are represented by white squares. Marker-based-selection-derived hybrids are represented by black squares. Conventional-breeding-derived hybrids are represented by crosses. The hybrids that show high grain yield and low grain moisture at harvest are positioned in the upper left corner of Figure 7.

DETAILED DESCRIPTION

The presently disclosed subject matter relates to virtually (theoretically) deriving the progeny of interest (through modeling of selfing, crossing, or combinations thereof) and computing their probabilities of occurrence and their genome-wide genetic values. The presently disclosed subject matter can consider, in some embodiments, the entire genome simultaneously, thereby taking into account linkage disequilibrium and leading to realistic predictions.

As such, the presently disclosed subject matter can provide for the development of more efficient marker- and/or QTL-based breeding than existing technologies.

The presently disclosed subject matter relates in some embodiments to selecting individuals (e.g., plants) or groups (e.g., pairs) of individuals based on the genetic values and genetic characteristics of their progeny, rather than on their own genetic values and genetic characteristics. In some embodiments, progeny are not actually derived and assessed but only "theoretically" derived through analytical computations (exact or approximate) or simulations. Based on these "theoretical" genetic values, progeny may or may not be actually derived (as desired) through specific breeding schemes (including, but not limited to selfing, crossing, and combinations thereof). Genetic values and characteristics of the progeny depend on the genetic characteristics of their parents after the action of meiosis and fertilization. The presently disclosed subject matter relates to calculating and/or simulating how genetic characteristics of individuals pass meiosis and fertilization to create new individuals (progeny), and assessing genome-wide genetic values of these progeny. In some embodiments, calculations and/or simulations can take into account genetic markers and all linkages between them, as well as the characteristics of the associations between genetic markers and phenotypic traits.

## 1. Definitions

Unless defined otherwise, all technical and scientific terms used herein have the same meaning as commonly understood by one of ordinary skill in the art to which the presently disclosed subject matter pertains. The following definitions supplement those in the art and are directed to the current application and are not to be imputed to any related or unrelated case; e.g., to any commonly owned patent or application. Although any methods and materials similar or equivalent to those described herein can be used in the practice for testing of the presently disclosed subject matter, exemplary materials and methods are described herein. Accordingly, the terminology used herein is for the purpose of describing particular embodiments only, and is not intended to be limiting.

As used in this specification and the appended claims, the singular forms "a", "an", and "the" include plural referents unless the context clearly dictates otherwise. Thus, for example, reference to "a protein" includes one or more proteins, and reference to "a cell" includes mixtures of cells, tissues, and the like.

As used herein, the terms "allele" and "allelic variant" refer to any of one or more alternative forms of a gene or genetic marker. In a diploid cell or organism, the two alleles of a given gene (or marker) typically occupy corresponding loci on a pair of homologous chromosomes.

As used herein, the terms "association", "associated with", and grammatical variants thereof refer to a definable relationship between two or more entities. The relationship can be of any type and scope based on the nature of the entities and the context in which the terms appear.

For example, a genotype can be associated with a probability of occurrence or a frequency of occurrence. This usage refers to the fact that a probability or a frequency of occurrence of a particular genotype can be calculated and/or otherwise determined based on knowledge, testing, calculation, simulation, or any other manipulation of other genotypes that are related to the particular genotype as parent, sib, or progeny. The fact that the probability of occurrence or the frequency of occurrence of the particular genotype can be determined from the other genotypes means that there is an association (*i.e.*, a relationship) between the various genotypes.

Similarly, each genotype can be associated with a genetic value. In some embodiments, a genotype is associated with a genetic value when one or more alleles that comprise the genotype are assigned a genetic value and the genetic values so assigned are summed or otherwise calculated for each individual allele that makes up the genotype to arrive at a genetic value for the genotype as a whole. Although the genetic values that are assigned to each allele can be assigned based on whatever criteria the assignor deems important, once genetic values are assigned to one or more alleles, a given genotype that is made up of combinations of these alleles will have a specific genetic value based on the individual genetic values so assigned. Thus, a genotype can be considered to be associated with a genetic value based on the calculation employed for the individual alleles.

A genetic locus can also be associated with expression of a phenotypic trait. In this context, the genetic locus is understood to influence the expression of the phenotypic trait. Stated another way, a genetic locus that is associated with expression of a phenotypic trait is a locus (*e.g.*, a QTL) for which the various alleles that can be present at that locus affect some aspect of the phenotype. Similarly, associations can exist between genetic markers and phenotypic traits, particularly when the presence of a genetic marker is indicative and/or predictive of the presence of an allele that itself is associated with expression of the phenotypic trait.

As used herein, the term "breeding", and grammatical variants thereof, refer to any process that generates a progeny individual. Breedings can be sexual or asexual, or any combination thereof. Exemplary non-limiting types of breedings include crossings, selfings, doubled haploid derivative generation, and combinations thereof. As disclosed herein, these breedings need not be performed to generate physical progeny, but can be modeled using, for example, the predictive calculations and/or simulations disclosed herein.

As used herein, the phrase "diploid individual" refers to an individual that has two sets of chromosomes, typically one from each of its two parents. However, it is understood that in some embodiments a diploid individual can receive its "maternal" and "paternal" sets of chromosomes from the same single organism, such as when a plant is selfed to produce a subsequent generation of plants.

As used herein, the phrase "established breeding population" refers to a collection of potential breeding partners produced by and/or used as parents in a breeding program; e.g., a commercial breeding program. The members of the established breeding population are typically well-characterized genetically and/or phenotypically. For example, several phenotypic traits of interest might have been evaluated, e.g., under different environmental conditions, at multiple locations, and/or at different times. Alternatively or in addition, one or more genetic loci associated with expression of the phenotypic traits might have been identified and one or more of the members of the breeding population might have been genotyped with respect to the one or more genetic loci as well as with respect to one or more genetic markers that are associated with the one or more genetic loci.

As used herein, the term "$F_0$" refers to an initial individual or plurality of individuals (*e.g.,* a first and a second breeding partner) that are used to generate the subsequent generations as set forth herein. It is noted that while an $F_0$ individual is in some embodiments an inbred individual and thus additional genetically identical individuals exist, it is not necessary that this be the case. In some embodiments, therefore, the term "$F_0$" is a relative term that is employed herein to refer to an individual or plurality of individuals that are bred or that otherwise donate genetic information to subsequent generations (e.g., $F_1$, $F_2$, $F_3$, $F_{n-1}$, $F_n$, etc.). Thus, as used herein, $F_0$ can in some embodiments refer to an individual of a generation that produces an $F_1$ generation, even if there are one or more generations that actually precede the generation of which the designated $F_0$ individual is a member.

As used herein, the term "$F_1$" refers to the first filial generation, the progeny of a breeding between, for example, two $F_0$ individuals (e.g., a first and a second breeding partner) or between two $F_0$ inbred lines as defined herein. It is also possible to generate an $F_1$ individual or generation by selfing an $F_0$ individual or by othertechniques that are known in the art of husbandry. As used herein, the term "advanced generation" refers to the second and subsequent filial generations (e.g., the $F_2$, $F_3$, and later generations) produced from the $F_1$ progeny by selfing or sexual crosses (e.g., with other $F_1$ progeny, with an inbred line, etc.).

As used herein, the term "founder" refers to an inbred or single cross $F_1$ hybrid that contains one or more alleles (e.g., genetic marker alleles) that can be tracked through the founder's descendents in a pedigree of a population; e.g., a breeding population. In an established breeding population, for example, the founders are typically (but not necessarily) the earliest developed lines.

As used herein, the term "gene" is used broadly to refer to any nucleic acid associated with a biological function. Genes typically include coding sequences and/or regulatory sequences required for expression of such coding sequences.

As used herein, the phrase "genetic marker" refers to a feature of an individual's genome (e.g., a nucleotide or a polynucleotide sequence that is present in an individual's genome) that is associated with one or more loci of interest. In some embodiments, a genetic marker is polymorphic in a population of interest, or the locus occupied by the polymorphism, depending on context. Genetic markers include, for example, single nucleotide polymorphisms (SNPs), indels (i.e., insertions/deletions), simple sequence repeats (SSRs), restriction fragment length polymorphisms (RFLPs), random amplified polymorphic DNAs (RAPDs), cleaved amplified polymorphic sequence (CAPS) markers, Diversity Arrays Technology (DArT) markers, and amplified fragment length polymorphisms (AFLPs), among many other examples. Genetic markers can, for example, be used to locate genetic loci containing alleles that contribute to variability in expression of phenotypic traits on a chromosome. The phrase "genetic marker" can also refer to a polynucleotide sequence complementary to a genomic sequence, such as a sequence of a nucleic acid used as probes.

A genetic marker can be physically located in a position on a chromosome that is within or outside of to the genetic locus with which it is associated (i.e., is intragenic or extragenic, respectively). Stated another way, whereas genetic markers are typically employed when the location on a chromosome of the gene that corresponds to the locus of interest has not been identified and there is a non-zero rate of recombination between the genetic marker and the locus of interest, the presently disclosed subject matter can also employ genetic markers that are physically within the boundaries of a genetic locus (e.g., inside a genomic sequence that corresponds to a gene such as, but not limited to a polymorphism within an intron or an exon of a gene). In some embodiments of the presently disclosed subject matter, the one or more genetic markers comprise between one and ten markers, and in some embodiments the one or more genetic markers comprise more than ten genetic markers.

As used herein, the term "genotype" refers to the genetic constitution of a cell or organism. An individual's "genotype for a set of genetic markers" includes the specific alleles, for one or more genetic marker loci, present in the individual. As is known in the art, a genotype can relate to a single locus or to multiple loci, whether the loci are related or unrelated and/or are linked or unlinked. In some embodiments, an individual's genotype relates to one or more genes that are related in that the one or more of the genes are involved in the expression of a phenotype of interest (e.g., a quantitative trait as defined herein). Thus, in some embodiments a genotype comprises a summary of one or more alleles present within an individual at one or more genetic loci of a quantitative trait. In some embodiments, a genotype is expressed in terms of a haplotype (defined herein below).

As used herein, the term "germplasm" refers to the totality of the genotypes of a population or other group of individuals (e.g., a species). The term "germplasm" can also refer to plant material; e.g., a group of plants that act as a repository for various alleles. The phrase "adapted germplasm" refers to plant materials of proven genetic superiority; e.g., for a given environment or geographical area, while the phrases "non-adapted germplasm," "raw germplasm," and "exotic germplasm" refer to plant materials of unknown or unproven genetic value; e.g., for a given environment or geographical area; as such, the phrase "non-adapted germplasm" refers in some embodiments to plant materials that are not part of an established breeding population and that do not have a known relationship to a member of the established breeding population.

As used herein, the term "haplotype" refers to the set of alleles an individual inherited from one parent. A diploid individual thus has two haplotypes. The term "haplotype" can be used in a more limited sense to refer to physically linked and/or unlinked genetic markers (e.g., sequence polymorphisms) associated with a phenotypic trait. The phrase "haplotype block" (sometimes also referred to in the literature simply as a haplotype) refers to a group of two or more genetic markers that are physically linked on a single chromosome (or a portion thereof). Typically, each block has a few common haplotypes, and a subset of the genetic markers (i.e., a "haplotype tag") can be chosen that uniquely identifies each of these haplotypes.

The phrase "high throughput screening" refers to assays in which the format allows large numbers of samples to be screened. In some embodiments, the phrase "high throughput screening" refers to assays in which the format allows large numbers of genetic markers (e.g., nucleic acid sequences), large numbers of individual or pools of genotypes, or both, to be screened. In the context of the presently disclosed subject matter, the phrase "high throughput screening" refers in some embodiments to the screening of large numbers of genotypes as individuals or pools for nucleic acid sequences of the genome of an individual to identify the presence of genetic marker alleles.

As used herein, the terms "hybrid", "hybrid plant," and "hybrid progeny" refers to an individual produced from genetically different parents (e.g., a genetically heterozygous or mostly heterozygous individual).

If two individuals possess the same allele at a particular locus, the alleles are termed "identical by descent" if the alleles were inherited from one common ancestor (i.e., the alleles are copies of the same parental allele). The alternative is that the alleles are "identical by state" (i.e., the alleles appear the same but are derived from two different copies of the allele). Identity by descent information is useful for linkage studies; both identity by descent and identity by state information can be used in association studies such as those described herein, although identity by descent information can be particularly useful.

As used herein, the phrase "inbred line" refers to a genetically homozygous or nearly homozygous population. An inbred line, for example, can be derived through several cycles of brother/sister breedings or of selfing. In some embodiments, inbred lines breed true for one or more phenotypic traits of interest. An "inbred", "inbred individual", or "inbred progeny" is an individual sampled from an inbred line.

As used herein, the term "linkage", and grammatical variants thereof, refers to the tendency of alleles at different loci on the same chromosome to segregate together more often than would be expected by chance if their transmission were independent, in some embodiments as a consequence of their physical proximity.

As used herein, the phrase "linkage disequilibrium" (also called "allelic association") refers to a phenomenon wherein particular alleles at two or more loci tend to remain together in linkage groups when segregating from parents to offspring with a greater frequency than expected from their individual frequencies in a given population. For example, a genetic marker allele and a QTL allele can show linkage disequilibrium when they occur together with frequencies greater than those predicted from the individual allele frequencies. Linkage disequilibrium can occur for several reasons including, but not limited to the alleles being in close proximity on a chromosome

As used herein, the term "locus" refers to a position on a chromosome (e.g., of a gene, a genetic marker, or the like).

As used herein, the phrase "nucleic acid" refers to any physical string of monomer units that can be corresponded to a string of nucleotides, including a polymer of nucleotides (e.g., a typical DNA or RNA polymer), modified oligonucleotides (e.g., oligonucleotides comprising bases that are not typical to biological RNA or DNA, such as 2'-O-methylated oligonucleotides), and the like. In some embodiments, a nucleic acid can be single-stranded, double-stranded, multi-stranded, or combinations thereof. Unless otherwise indicated, a particular nucleic acid sequence of the presently disclosed subject matter optionally comprises or encodes complementary sequences, in addition to any sequence explicitly indicated.

As used herein, the phrase "phenotypic trait" refers to the appearance or other detectable characteristic of an individual, resulting from the interaction of its genome with the environment.

As used herein, the term "plurality" refers to more than one. Thus, a "plurality of individuals" refers to at least two individuals. In some embodiments, the term plurality refers to more than half of the whole. For example, in some embodiments a "plurality of a population" refers to more than half the members of that population.

As used herein, the term "progeny" refers to the descendant(s) of a particular cross. Typically, progeny result from breeding of two individuals, although some species (particularly some plants and hermaphroditic animals) can be selfed (i.e., the same plant acts as the donor of both male and female gametes). The descendant(s) can be, for example, of the $F_1$, the $F_2$, or any subsequent generation.

As used herein, the phrase "qualitative trait" refers to a phenotypic trait that is controlled by one or a few genes that exhibit major phenotypic effects. Because of this, qualitative traits are typically simply inherited. Examples in plants include, but are not limited to, flower color, cob color, and disease resistance such as Northern corn leaf blight resistance.

As used herein, the term "quantile" refers to a point along a probability or frequency curve below which a desired percentage of the events fall. For example, the "50% quantile" corresponds to that point on a probability or frequency curve below which 50% of the events fall. Similarly, the "95% quantile" corresponds to that point on a probability or frequency curve below which 95% of the events fall. In some embodiments, a 50% quantile or a 95% quantile relates to that point on a plot of genetic values versus probability or frequency of occurrence as calculated, simulated, or combinations of calculated and simulated using the presently disclosed methods that is greater than 50% or 95%, respectively, of the possible genetic values that can be generated by the calculating, simulating, or combinations of calculating and simulating. In some embodiments, a 50% quantile or a 95% quantile relates to the genetic value that corresponds to that point on a plot of genetic values versus probability or frequency of occurrence as calculated, simulated, or combinations of calculated and simulated using the presently disclosed methods that is greater than 50% or 95%, respectively, of the possible genetic values that can be generated by the calculating, simulating, or combinations of calculating and simulating.

As used herein, the phrase "quantitative trait" refers to a phenotypic trait that can be described numerically (*i.e.,* quantitated or quantified). A quantitative trait typically exhibits continuous variation between individuals of a population; that is, differences in the numerical value of the phenotypic trait are slight and grade into each other. Frequently, the frequency distribution in a population of a quantitative phenotypic trait exhibits a bell-shaped curve (i.e., exhibits a normal distribution between two extremes). A quantitative trait is typically the result of a genetic locus interacting with the environment or of multiple genetic loci (QTL) interacting with each other and/or with the environment. Examples of quantitative traits include plant height and yield.

As used herein, the terms "quantitative trait locus" (QTL) and "marker trait association" refer to an association between a genetic marker and a chromosomal region and/or gene that affects the phenotype of a trait of interest. Typically, this is determined statistically; *e.g.,* based on one or more methods published in the literature. A QTL can be a chromosomal region and/or a genetic locus with at least two alleles that differentially affect the expression of a phenotypic trait (either a quantitative trait or a qualitative trait).

As used herein, the phrases "sexually crossed" and "sexual reproduction" in the context of the presently disclosed subject matter refers to the fusion of gametes to produce progeny (e.g., by fertilization, such as to produce seed by pollination in plants). A "sexual cross" or "cross-fertilization" is in some embodiments fertilization of one individual by another (e.g., cross-pollination in plants). The term "selfing" refers in some embodiments to the production of seed by self-fertilization or self-pollination; i.e., pollen and ovule are from the same plant.

As used herein, the phrase "single cross $F_1$ hybrid" refers to an $F_1$ hybrid produced from a cross between two inbred lines.

As used herein, the term "tester" refers to a line or individual with a standard genotype, known characteristics, and established performance. A "tester parent" is an individual from a tester line that is used as a parent in a sexual cross. Typically, the tester parent is unrelated to and genetically different from the individual to which it is crossed. A tester is typically used to generate $F_1$ progeny when crossed to individuals or inbred lines for phenotypic evaluation.

As used herein, the phrase "topcross combination" refers to the process of crossing a single tester line to multiple lines. The purpose of producing such crosses is to determine phenotypic performance of hybrid progeny; that is, to evaluate the ability of each of the multiple lines to produce desirable phenotypes in hybrid progeny derived from the line by the tester cross.

As used herein, the term "transgenic" refers to a cell or an individual into which one or more exogenous polynucleotides have been introduced by any technique other than sexual cross or selfing. Examples of techniques by which this can be accomplished are known in the art. In some embodiments, a transgenic individual is a transgenic plant, and the technique employed to create the transgenic plant is selected from the group consisting of *Agrobacterium*-mediated transformation, biolistic methods, electroporation, *in planta* techniques, and the like. Transgenic individuals can also arise from sexual crosses or by selfing of transgenic individuals into which exogenous polynucleotides have been introduced.

## II. Methods for Calculating a Distribution of a Probability or Frequency of Occurrence of One or More Potential Genotypes

In some embodiments, the presently disclosed subject matter provides methods for calculating a distribution of a probability or frequency of occurrence of one or more potential genotypes. In some embodiments, the methods comprise (a) providing a first breeding partner and a second breeding partner, wherein (i) the genotype of each of the first breeding partner and the second breeding partner is known or is predictable with respect to one or more genetic markers, each of which is linked to a genetic locus; and (ii) a genetic distance between each genetic marker and the genetic locus to which it is linked is known or can be assigned; (b) calculating, simulating, or combinations of calculating and simulating a breeding of the first breeding partner and the second breeding partner to generate a subsequent generation, each member of the subsequent generation comprising a genotype; and (c) calculating a distribution of a probability or a frequency of occurrence for one or more of the genotypes of one or more members of the subsequent generation.

As used herein, the phrase "calculating a distribution of a probability or frequency of occurrence of one or more potential genotypes" refers to methods for generating probabilities and/or frequencies of occurrence for one or more genotypes that can be produced when an individual with a known or predictable genotype is selfed, crossed to another individual with a known or predictable genotype, or generated by calculating or simulating a doubled haploid breeding of an individual from a prior generation (e.g., from the immediately prior generation). In some embodiments, the phrase refers to methods for generating probabilities and/or frequencies of occurrence for all possible genotypes that can be produced when an individual with a known or predictable genotype is selfed, crossed to another individual with a known or predictable genotype, or generated by calculating or simulating a doubled haploid breeding of an individual from a prior generation (e.g., from the immediately prior generation).

Thus, in some embodiments the phrase refers to determining all or a subset of all potential genotypes that can be produced when a progeny individual is produced from one or more known or predictable genotypes as well as determining an expected probability and/or frequency at which each such genotype would be expected to occur.

As used herein, the phrase "known" in the context of a genotype of an individual with respect to one or more genetic markers refers to a genotype for which the presence or absence and/or the identity of the one or more genetic markers has been ascertained for an individual (e.g., has been determined experimentally or otherwise). The phrase "predictable" in the context of a genotype of an individual with respect to one or more genetic markers refers to a genotype for which the presence or absence and/or the identity of the one or more genetic markers can be calculated or otherwise predicted for an individual, for example by comparison to one or more related individuals (e.g., progenitors or offspring of any generation) for which the genotypes are known. For example, when the genotypes of the parents of an individual are known, it is possible to predict the possible genotypes that the individual can have, along with the probability or frequency at which each such possible genotype can occur. Therefore, a genotype with respect to one or more genetic markers is deemed to be predictable when the genotype of the individual can be determined with reference to the genotypes of one or more progenitors and/or one or more progeny, with either or both of the progenitors and progeny being 1, 2, or more generations removed from the individual itself.

In some embodiments of the presently disclosed methods, a genetic distance between each genetic marker and the genetic locus to which it is linked is known or can be assigned. As used herein, the phrase "genetic distance" refers to an absolute or a relative distance between a genetic marker and a genetic locus to which it is associated. In some embodiments, a genetic distance is a physical distance, and can be expressed in term such as, but not limited to, bases, kilobases, megabases, etc. In some embodiments, a genetic distance is a relative distance, and can be expressed in terms such as, but not limited to, a recombination rate between the genetic marker and the genetic locus. Terms that can be employed to express genetic distances that are based on recombination rates include, but are not limited to percent recombination and its associated term centiMorgan (cM). It is understood that recombination occurs at different rates or frequencies in different species and also in different regions of different chromosomes in the same species, and thus a centiMorgan can refer to a different absolute number of bases in different contexts.

In the presently disclosed methods, genetic distances between genetic markers and genetic loci can be known or can be assigned. When a genetic distance is "known", it has been determined experimentally to have a particular value. When a genetic distance can be "assigned", it may not have been precisely determined experimentally, but can be predicted based on whatever information might be available.

As used herein, the terms "first breeding partner" and "second breeding partner" refer to any individuals that can provide male gametes and female gametes. Accordingly, in some embodiments the first breeding partner and the second breeding partner can be different members of the same species.

The individuals that comprise the breeding partners, the breeding pairs, and the progeny can be of any species. In some embodiments, each breeding partner is a plant. Any plant species can be employed. In some embodiments, the plant is selected from the group consisting of maize, wheat, barley, rice, sugar beet, sunflower, winter oilseed rape, canola, tomato, pepper, melon, watermelon, broccoli, cauliflower, Brussel sprouts, lettuce, spinach, sugar cane, coffee, cocoa, pine, poplar, eucalyptus, apple tree, and grape. In some embodiments, the plant is a maize plant.

Additionally, the individuals that comprise the breeding partners, the breeding pairs, and the progeny can be inbred or outbred. In some embodiments, the individuals that comprise the breeding partners, the breeding pairs, and the progeny are inbred individuals or are the $F_1$ progeny of one or two inbred individuals.

In some embodiments, the species is one that can be bred by selfing. Therefore, in these embodiments the first and the second breeding partners can be the same individual. In some embodiments, the future generation is generated by at least two successive generations of selfing of one or more members of a preceding generation. In some embodiments, the future generation is generated by three successive generations of selfing of one or more members of a preceding generation. In some embodiments, the future generation is generated by four successive generations of selfing of one or more members of a preceding generation.

In some embodiments, the presently disclosed methods employ doubled haploid derivatives of an individual of a previous generation. Doubled haploid derivatives of an individual are produced by the doubling of a set of chromosomes (1 N) from a heterozygous plant to produce a completely homozygous individual. Methods for producing doubled haploid derivatives are known in the art (see *e.g.,* Wan *et al.,* 1989; U.S. Application Publication No. 20030005479; U.S. Patent No. 7,135,615). This can be advantageous because the process omits the generations of selfing needed to obtain a homozygous plant from a heterozygous source.

In some embodiments of the presently disclosed methods, (i) the genotype of each of the first breeding partner and the second breeding partner is known or is predictable with respect to one or more genetic markers, each of which is linked to a genetic locus; and (ii) a genetic distance between each genetic marker and the genetic locus to which it is linked is known or can be assigned. Methods for genotyping individuals with respect to one or more genetic loci are known, as are methods for identifying distances between genetic markers and genetic loci to which the markers are linked. Disclosed herein below are strategies whereby this information can be employed for calculating and/or predicting a distribution of a probability or a frequency of occurrence of one or more potential genotypes in a subsequent generation based on simulated and/or calculated breedings between the first and second breeding partners and subsequently their simulated and/or calculated progeny.

In some embodiments, the one or more genetic markers are selected from the group consisting of a single nucleotide polymorphism (SNP), an indel (i.e., insertion/deletion), a simple sequence repeat (SSR), a restriction fragment length polymorphism (RFLP), a random amplified polymorphic DNA (RAPD), a cleaved amplified polymorphic sequence (CAPS) marker, a Diversity Arrays Technology (DArT) marker, an amplified fragment length polymorphism (AFLP), and combinations thereof. In some embodiments, the one or more genetic markers comprise between one and ten markers, and in some embodiments the one or more genetic markers comprise more than ten genetic markers.

In some embodiments, the calculating, simulating, or combinations of calculating and simulating a breeding includes calculating, simulating, or combinations of calculating and simulating an expected rate of recombination between at least one of the one or more genetic markers and a genetic locus associated with expression of a phenotypic trait. A representative method for calculating, simulating, or combinations of calculating and simulating an expected rate of recombination between at least one of the one or more genetic markers and a genetic locus associated with expression of a phenotypic trait is set forth hereinbelow.

In some embodiments, the phenotypic trait is a quantitative trait, and in some embodiments the one or more genetic markers are linked to one or more quantitative trait loci associated with expression of the phenotypic trait. In some embodiments, the genetic locus associated with expression of the phenotypic trait encodes a gene product that is associated with expression of the phenotypic trait. In some embodiments, the rate of recombination between the at least one of the one or more genetic markers and the genetic locus associated with expression of the phenotypic trait is zero.

The presently disclosed methods employ calculating, simulating, or combinations of calculating and simulating of a breeding of the first breeding partner and the second breeding partner to generate a subsequent generation. As used herein, the phrase "subsequent generation" refers to a generation of one or more progeny that results from the calculated, simulated, or combinations of both calculated and simulated breeding of the first breeding partner and the second breeding partner. Thus, if the first and second breeding partners are arbitrarily assigned to be the $F_0$ generation, then the members of the "subsequent generation" are the $F_1$ generation.

This is to be contrasted with the "further generation", which in the context of the presently disclosed subject matter refers to any generation that follows the "subsequent generation". Stated another way, the first and second breeding partners can be assigned to be the $F_0$ generation, which are then bred by calculating, simulating, or combinations of calculating and simulating a breeding to produce an $F_1$ generation that is referred to herein as the "subsequent generation", individuals of which can optionally be bred for one or more additional generations to produce one or more "further generations" (*i.e.*, the $F_2$, $F_3$, $F_4$, $F_5$, ..., $F_n$ generations).

II.A. Representative Approaches for Calculating a Probability or a Frequency Distribution

Given that it is within the scope of the presently disclosed subject matter to employ a subsequent generation and optionally any number of further generations, and further that the breedings that are calculated and/or simulated can include breedings of any combinations of individuals from any of these generations as well as derivatives thereof (e.g., doubled haploid derivatives), there can be many potential genotypes that can exist in the members of the subsequent and further generations. In some embodiments, the presently disclosed methods comprise calculating a distribution of a probability or frequency of occurrence of one or more of the potential genotypes that can be calculated.

Thus, in some embodiments the presently disclosed subject matter provides methods that relate to calculating and/or predicting a distribution of a probability or a frequency of occurrence of one or more potential genotypes. In some embodiments, the distribution of a probability or a frequency of occurrence of one or more potential genotypes relates to a distribution of a probability or a frequency of occurrence of one or more potential genotypes in a progeny individual based on knowledge of parental genotypes (*i.e.*, the first breeding partner and the second breeding partner, which in some embodiments are the same individual such as when plants are selfed).

II.A.1. Generally

A genotype can be considered and assigned the symbol $_{t-1}^{w}[G]_{ij}$. The lower left index refers to the generation, the upper left one to the parent type (w = 1,2), and the lower right to the upper and lower haplotype indexes, respectively. This genotype is described as the pairing of two chromosomes: chromosomes are assumed with $L$ loci and a chromosome is represented by a vector |g with $L$ components taking binary values on $\{0,1\}$. Symbol o represents the ordered (from top to bottom) pairing operator of two chromosomes. Taking all this into account, genotypes $_{t-1}^{1}[G]_{ij}$ and $_{t-1}^{2}[G]_{ij}$ can be written as:

$$_{t-1}^{1}[G]_{ij} = {}_{t-1}^{w_1}|g\rangle_i \; o \; {}_{t-1}^{w_1{}'}|g\rangle_j$$

$$_{t-1}^{2}[G]_{ij} = {}_{t-1}^{w_2}|g\rangle_i \; o \; {}_{t-1}^{w_2{}'}|g\rangle_j \,,$$

where $w$ and $w'$ are the indexes of the parents who generated these gametes; they are linked by the relation: $w + w' = 3$.

The steps for recombination, segregation, and then fertilization are then considered by writing each time the associated probability densities.

II.A.2. Recombination

$_{t-1}[\tilde{G}]_{mn}$ designates the genotype obtained after recombination operation on genotype $_{t-1}[G]_{ij}$. The event probability is then:

$$Pr\left\{_{t-1}[\tilde{G}]_{mn}\right\} = \sum_{i=1}^{N}\sum_{j=1}^{N} Pr\left\{_{t-1}[\tilde{G}]_{mn} \; \middle| \; _{t-1}[G]_{ij}\right\} Pr\left\{_{t-1}[G]_{ij}\right\} \,,$$

where the writing $Pr\{x|y\}$ expresses the occurrence probability of event $y$ conditioned to event $x$. The above summation is carried out on the entire genetic space; i.e., $N \times N = 2^L \times 2^L$ states. In fact, taking into account the genetic equivalence of $(i, j)$ and $(j,i)$ couples, the number of distinct states is reduced to $\dfrac{2^L\left(2^L+1\right)}{2}$.

II.A.3. Segregation

The expression of the generation probability of a gamete $_{t-1}^{w}|g\rangle$ with the segregation process of the genotypes $_{t-1}^{w}[\tilde{G}]_{mn}$ is:

$$Pr\left\{_{t-1}^{w}|g\rangle\right\} = \sum_{m=1}^{N}\sum_{n=1}^{N} Pr\left\{_{t-1}^{w}|g\rangle \; \middle| \; _{t-1}^{w}[\tilde{G}]_{mn}\right\} Pr\left\{_{t-1}^{w}[\tilde{G}]_{mn}\right\}$$

In order to express the conditional probability, it was chosen that the segregation is limited to freeing the upper haplotype. This segregation choice is mathematically translated by the expression:

$$Pr\left\{ \left. _{t-1}^{w}|g\rangle \right| \ _{t-1}^{w}\left[\tilde{G}\right]_{mn} \right\} = F\left\{ _{t-1}^{w}|g\rangle - _{t-1}^{w}|g\rangle_{m} \right\},$$

where the function $F(|x-|x_0)$ is defined by:

$$F\left(|x\rangle - |x\rangle_0 \right) = 1 \text{ for } |x\rangle = |x\rangle_0$$

$$F\left(|x\rangle - |x\rangle_0 \right) = 0 \text{ for } |x\rangle \neq |x\rangle_0$$

This choice is entirely compatible with a recombination operation accepting the chromosome interverting of a genotype. For this exchange to be possible with a ½ occurrence, all it takes is to allow a recombination between loci **0** and **1** to occur with a ½ probability.

Injecting this conditional probability expression into the probability $Pr\left\{ _{t-1}^{w}|g\rangle \right\}$ generates the expression:

$$Pr\left\{ _{t-1}^{w}|g\rangle_u \right\} = \sum_{n=1}^{N} Pr\left\{ _{t-1}^{w}\left[\tilde{G}\right]_{un} \right\}$$

This expresses a gamete marginal probability of occurrence; this result can be extended to the compound probability of the ranked juxtaposition: $_{t-1}^{w}|g\rangle_u \ \& \ _{t-1}^{w'}|g\rangle_v$ :

$$Pr\left\{ _{t-1}^{w}|g\rangle_u \ \& \ _{t-1}^{w'}|g\rangle_v \right\} = \sum_{m=1}^{N}\sum_{n=1}^{N} Pr\left\{ _{t-1}^{w}\left[\tilde{G}\right]_{um} \ \& \ _{t-1}^{w'}\left[\tilde{G}\right]_{vn} \right\}$$

II.A.4. <u>Fertilization</u>

Finally, the probability for creating the genotype, $[G]_{uv} = {}_{t-1}|g_u O_{t-1}|g_v$ from fertilization can be determined. Fertilization will assemble gametes $_{t-1}^{w}|g\rangle_u$ and $_{t-1}^{w'}|g\rangle_v$ under the constraint **w + w' = 3**. The construction probability of genotype $_t[G]_{uv}$ is therefore equal to:

$$Pr\left\{ _t[G]_{uv} \right\} = \frac{1}{2}\sum_{w=1}^{2}\sum_{w'=1}^{2} Pr\left\{ _{t-1}^{w}|g\rangle_u \ O \ _{t-1}^{w'}|g\rangle_v \right\} F\left\{ w + w' - 3 \right\},$$

where the factor ½ expresses the probability of a given ordering (in terms of parents' type).

As set forth hereinabove, the probability $Pr\left\{ _{t-1}^{w}|g\rangle_u O _{t-1}^{w'}|g\rangle_v \right\}$ develops into:

$$Pr\left\{ {}_{t-1}^{w}|g\rangle_u \circ {}_{t-1}^{w'}|g\rangle_v \right\} = \sum_{m=1}^{N}\sum_{n=1}^{N} Pr\left\{ {}_{t-1}^{w}\left[\tilde{G}\right]_{um} \& {}_{t-1}^{w'}\left[\tilde{G}\right]_{vn} \right\}$$

Using the total probabilities theorem, the probability of event ${}_{t-1}^{w}\left[\tilde{G}\right]_{um}$ & ${}_{t-1}^{w'}\left[\tilde{G}\right]_{vm}$ can be developed into the sum below:

$$Pr\left\{ {}_{t-1}^{w}\left[\tilde{G}\right]_{um} \& {}_{t-1}^{w'}\left[\tilde{G}\right]_{vn} \right\} = \frac{1}{2}$$
$$\sum_{i=1}^{N}\sum_{j=1}^{N}\sum_{i'=1}^{N}\sum_{j'=1}^{N} Pr\left\{ \left[ {}_{t-1}^{w}\left[\tilde{G}\right]_{um} \& {}_{t-1}^{w'}\left[\tilde{G}\right]_{vn} \right] \Big| \left[ {}_{t-1}^{w}\left[G\right]_{ij} \& {}_{t-1}^{w'}\left[G\right]_{i'j'} \right] \right\}$$
$$Pr\left\{ {}_{t-1}^{w}\left[G\right]_{ij} \& {}_{t-1}^{w'}\left[G\right]_{i'j'} \right\}$$

Taking into account the independence of recombination events, the conditional probability can be factorized as:

$$Pr\left\{ \left[ {}_{t-1}^{w}\left[\tilde{G}\right]_{um} \& {}_{t-1}^{w'}\left[\tilde{G}\right]_{vn} \right] \Big| \left[ {}_{t-1}^{w}\left[\tilde{G}\right]_{ij} \& {}_{t-1}^{w'}\left[\tilde{G}\right]_{i'j'} \right] \right\} =$$
$$Pr\left\{ {}_{t-1}^{w}\left[\tilde{G}\right]_{um} \Big| {}_{t-1}^{w}\left[\tilde{G}\right]_{ij} \right\} Pr\left\{ {}_{t-1}^{w'}\left[\tilde{G}\right]_{vn} \Big| {}_{t-1}^{w'}\left[\tilde{G}\right]_{i'j'} \right\}$$

All elements are now available for establishing the expression for the generation probability of a genotype ${}_{t}[G]_{uv}$ from the set of parental genotypes ${}_{t-1}^{w}\left[G\right]_{ij}$ , ${}_{t-1}^{3-w}\left[G\right]_{i'j'}$ with **w = 1,2**. The expression is thus:

$$Pr\left\{ {}_{t}[G]_{uv} \right\} = \frac{1}{2}\sum_{w=1}^{2}\sum_{m=1}^{N}\sum_{n=1}^{N}\sum_{i=1}^{N}\sum_{j=1}^{N}\sum_{i'=1}^{N}\sum_{j'=1}^{N} Pr\left\{ {}_{t-1}^{w}\left[\tilde{G}\right]_{um} \Big| {}_{t-1}^{w}\left[G\right]_{ij} \right\}$$
$$Pr\left\{ {}_{t-1}^{3-w}\left[\tilde{G}\right]_{vn} \Big| {}_{t-1}^{3-w}\left[G\right]_{i'j'} \right\} Pr\left\{ {}_{t-1}^{w}\left[G\right]_{ij} \& {}_{t-1}^{3-w}\left[G\right]_{i'j'} \right\}$$

II.B. Representative Approaches for Encoding the Genotype Information

The previous expression shows that the choice was made for the representation by two indexes. In fact, a genotype can be described by the indexes relative to the upper and lower chromosomes. However, the recombination simulation implicates both chromosomes simultaneously, and a more compact coding can be used for describing the couple state. The purpose of next section is to describe these coding modes, as well as the passage from one to the other.

II.B.1. The Various Coding Modes

As set forth hereinabove, genotype $[G]_{ij}$ is the ranked juxtaposition $|g_i \circ |g_j$, of two chromosomes, by convention the upper one being first, the lower one second. Since the coding requires their separate consideration, it is necessary to be able to differentiate them. Therefore, $|\hat{g}$ designates the upper haplotype, and $|\breve{g}$ the lower haplotype.

II.B.1.a. Coding by Two Vectors with Binary Elements

In a configuration having **L** loci, each $|g$ vector is the sequence of **L {0,1}** binary values. The coding of the genotype is therefore the juxtaposition of two vectors of this type.

II.B.1.b. Coding by Two Integer Values

The above binary coding can encode **N** = **2$^L$** possible states. An equivalent, but easier to handle because more compact, representation is that for two integers of the **{0,N-1}** domain which can be transformed in **{1,N}** domain index by adding the unit value. If the coding vector $|b_L$ is defined so that its **I** component equals:

$$\left| b_I \right\rangle_L = 2^{L\text{-}I}$$

the integer **i** = **C$_{ind}$** {$|g_i$} corresponding to the binary coding of haplotype $|g_i$ is the result of the scalar product:

$$i = {}_L\left\langle b \, | \, g \right\rangle + 1$$

II.B.1.c. Coding by a Unique Vector with Four-modality Elements

II.B.1.c.i.. Coding of States with Phases

A coding operator $\hat{C}_{pha}$, can be defined which, applied to a genotype, reduces the juxtaposition of both vectors to a single vector $|e$. This vector, which summarizes without information loss the genotype state, is obtained by the following operation:

$$\hat{C}_{pha} \left\{ [G] \right\} = \left| e \right\rangle$$

Coding of states with phases implies a four-modality coding for distinguishing types (0,0) and (1,1) homozygous states and of types (0,1) and (1,0) heterozygous states. Approaches for choosing this coding are disclosed herein.

II.B.1.c.ii. Coding of Experimental States

Coding operator $\hat{C}_{exp}$ is insensitive to the allele phase and generates a 3-modality coding for distinguishing types (0,0) and (1,1) homozygous states from heterozygous ones. It generates a vector $|\underline{e}$ using the operation:

$$\hat{C}_{exp} \left\{ [G] \right\} = \left| \underline{e} \right\rangle$$

An operator $\hat{T}$ can be defined such that **TC**$_{pha}$ = $\hat{C}_{exp}$, and allowing the passage:

$$\hat{T} \left| e \right\rangle = \left| \underline{e} \right\rangle$$

II.B.2. Choosing Codes

All degrees of freedom are available for choosing codes. In some embodiments, the simplicity of passing the code with phases over the experimental coding is employed. The following phenotype configuration:

$$\begin{matrix} a & A & a & A \\ a & a & A & A \end{matrix}$$

can be expressed as equivalent to the following binary coding:

$$\begin{matrix} 0 & 1 & 0 & 1 \\ 0 & 0 & 1 & 1 \end{matrix}$$

It can also be encoded with vector:

$$\begin{matrix} 0 & +1 & -1 & +2 \end{matrix}$$

This coding is obtained from relation:

$$e_l = + \hat{g}_l - \check{g}_l + 2\,\hat{g}_l\,\check{g}_l \,,$$

where $\hat{g}_l$ and $\check{g}_l$ as set forth hereinabove, are respectively the allele state coding vectors of the upper and lower haplotypes. The upper and lower chromosome coding can also be retrieved if the genotype coding is known by the relations:

$$\widehat{g}_l = \frac{1}{6}e_l\left[-2e_l^2 + 3e_l + 5\right]$$

$$\check{g}_l = \frac{1}{2}e_l\left[e_l - 1\right]$$

The passage to experimental coding is trivial since all it takes is the absolute value of the coding with phases:

$$\hat{T}\,|e\rangle = |\underline{e}\rangle = \big||e|\big\rangle$$

This experimental coding can be simply linked to the haplotype coding through:

$$\big||e|\big\rangle = |\check{g}\rangle + |\widehat{g}\rangle$$

II.C. Representative Approaches for Recombination Simulation

II.C.1. Recombination and Associated Probability

A vector $|\sigma$ is defined of length *L,* where the elements are binary values that contain the recombination information according to the following mode:

$\sigma_l = 1 \rightarrow$ Recombination between loci (**l-1**) and **l**.
$\sigma_l = 0 \rightarrow$ No recombination

On the other hand, also available is vector $|r$ where element $r_l$ is the recombination probability between loci (**l-1**) and **l**. At each configuration of vector $|\sigma$ corresponds a configuration of probability $\pi_{|\sigma}$ which expression is:

$$\pi_{|\sigma\rangle} = \prod_{l=1}^{L} (1 - r_l)^{(1-\sigma_l)} r_l^{\sigma_l}$$

In some embodiments, one of the roles assigned to the recombination process in the model is chromosome rank mixing before releasing the gametes. For this reason, in some embodiments: $r_l = \dfrac{1}{2}$. By adopting the principle according to which the recombination process includes the possibility of recombination before the first locus (with ½ probability), one degree of freedom can be added to the system. This results in a symmetry of the probability values while also making probable events $|\sigma^c$ and $|\overline{\sigma}^c$. Therefore, the recombinations identified by indexes $s =_L \langle b|\sigma^c + 1$ and $\overline{s} =_L \langle b|\overline{\sigma}^c + 1$ will be identical. While noting that $s + \overline{s} = N + 1$, this symmetry can be summarized as:

$$\pi_s = \pi_{N+l-s}$$

II.C.2. Description of State Changes

From vector $|\sigma$, a second vector $|\sigma^c$ is defined constructed according to the following cumulation procedure:

$$\sigma_v^c = 0 \text{ if sum } \sum_{v'=1}^{v} \sigma_{v'} \text{ is even}$$

$$\sigma_v^c = 1 \text{ if sum } \sum_{v'=1}^{v} \sigma_{v'} \text{ is odd}$$

Value **1** for locus **l** corresponds to an allele flip at the level of this locus, while value 0 refers to an unchanged situation, an even number of recombination between loci (**l - 1**) and **l** being without effect on the locus configuration.
A representative way to deduct vector $|\sigma$ from vector $|\sigma^c$ is to use the recurrence formula:

$$\mathbf{l = 1} \quad \sigma_l = \sigma_l^c$$

$$\mathbf{l > 1} \quad \sigma_l = \left(\sigma_l^c - \sigma_{l-1}^c\right)^2$$

II.D.. Representative Detailed Expression for Occurrence Probabilities Associated with Progeny

II.D.1. State Changes and Associated Probabilities

Concerning determining the probability

$$Pr\left\{\left|g\right\rangle_u o\left|g\right\rangle_v \middle| \left|g\right\rangle_i o\left|g\right\rangle_j\right\} = Pr\left\{ {}_{t-1}^w\left[\tilde{G}\right]_{uv} \middle| {}_{t-1}^w\left[G\right]_{ij} \right\}$$

from all recombination events, which, from a configuration $|g_i o|g_j$ ends up at configuration $|g_u o|g_v$. First, the required conditions to make the transition possible are considered. Second, the representation of the recombination event realizing this transition, as well as on establishing the expression of the associated probability, is considered.

II.D.1.a. Conditions for $(i, j) \leftrightarrows (u, v)$ Transfer

The conditions where a process or recombination allows the passage from a *(i,j)* state to a *(u,v)* state can be determined. These conditions can be established by using successively the three types of codes defined previously.

II.D.1.a.i. Binary Coding

The relationships describing the recombination action, but for each locus, can be set forth as:

- In the case where initial locus I is homozygous ($[\hat{g}_l]_i = [\breve{g}_l]_j$), in some embodiments the following equalities are satisfied:

$$\left[\widehat{g}_l\right]_u = \left[\breve{g}_l\right]_i$$

$$\left[\widehat{g}_l\right]_v = \left[\breve{g}_l\right]_j$$

This leads to:

$$\left[\widehat{g}_l\right]_u = \left[\breve{g}_l\right]_i = \left[\widehat{g}_l\right]_v = \left[\breve{g}_l\right]_j$$

Consequently, the state of the alleles for the homozygous loci must be the same for the two genotypes. The value of $\sigma_l^c$ is indifferent.

- In the case initial locus I is heterozygous ($[\hat{g}_l]_i = [\breve{g}_l]_j$), the component $\sigma_l^c$ can be obtained from the absolute value of the different states:

$$\sigma_l^c = \left|\left[\widehat{g}_l\right]_u - \left[\widehat{g}_l\right]_i\right|$$

In summary, in some embodiments the necessary and sufficient condition for realizing the translation is that the homologous loci must be homozygous and identical, or heterozygous.
If a heterozygous signature vector of $h_l$ element for each of these genotypes is defined:

$$\left|h\right\rangle_{ij} = \left|\left|g\right\rangle_i - \left|g\right\rangle_j\right|$$

$$\left| h \right\rangle_{uv} = \left\| g \right\rangle_u - \left| g \right\rangle_v \right|$$

and their complementary $|\bar{h}_{ij}$ and $|\bar{h}_{uv}$ vectors of $\bar{h}_l = l\text{-}h_l$ element, the constraints can be expressed by:

$$\left| h \right\rangle_{uv} = \left| h \right\rangle_{ij}$$

$$\left| \bar{h} \right\rangle_{uv} \cdot \left[ \left| g \right\rangle_u - \left| g \right\rangle_v \right] = \left| \bar{h} \right\rangle_{ij} \cdot \left[ \left| g \right\rangle_i - \left| g \right\rangle_j \right]$$

If a filtering function $f_{uv}^{ij}$ coded by value 1 is defined if the transition is feasible, 0 on the contrary, these constraints can be summarized by the expression:

$$f_{uv/ij} = F\left\{ \left| h \right\rangle_{uv} - \left| h \right\rangle_{ij} \right\} F\left\{ \left| \bar{h} \right\rangle_{uv} \cdot \left[ \left| g \right\rangle_u - \left| g \right\rangle_i \right] \right\}$$

II.D.1.a.ii. Coding with a Single Vector

Using codes $|e_{ij}$, in some embodiments the necessary and sufficient condition for a feasible transition *(ij)* $\leftrightarrows$ *(u,v)* by recombination is:

$$\left\| e \right\rangle_{uv} = \left\| e \right\rangle_{ij}$$

And therefore, in this system of representation, the filtering function is equal to:

$$f_{uv/ij} = F\left\{ \left\| e \right\rangle_{uv} - \left\| e \right\rangle_{ij} \right\}$$

II.D.1.a.iii. Coding with Two Indexes

In some embodiments, the $f_{uv/ij}$ filtration function expression established above can be used to derive the set of necessary and sufficient conditions for a feasible transition. By first considering the first factor, $F\{|h_{uv}\text{-}|h_{ij}\}$ imposing the same heterozygous signature, an index resulting from the binary code corresponding to this signature can be defined. If **H** is this index; it is then computed by:

$$H = {}_L\left\langle b \,|\, h \right\rangle + 1$$

A first condition is therefore expressed by:

$$H(i,j) = H(u,v)$$

Then, considering the second factor $F\{|\overline{h}_{uv}\cdot[|g_u-|g_i]\}$ imposing the identity of the homozygous loci, the integers corresponding to the homozygous part of each haplotype can be identical. If, on the other hand, the sum of the two integers corresponding to the heterozygous part of each haplotype is conserved by the recombination, the recombination operation conserves the sum of the indexes. The second condition can thus be expressed as:

$$u + v = i + j$$

In this way, the filtration function can be expressed as:

$$f_{uv/ij} = F\left\{H(u,v) - H(i,j)\right\} F\left\{u + v - i - j\right\}$$

II.D.1.b. Notion of Recombination Classes

Since the purpose of a filtration function such as the one just defined is to retain the couples compatible with the recombination, the notion of recombination classes can be derived. Such a class can be defined as a set of genotypes where each genotype *(i,j)* in this set can be linked to any other of this same set through a recombination operation. According to the expression of the filtering function which was just established, a class can be determined by indexes *H* and *S = i + j = u + v*. As for the individuals present in each class, they can be assigned through one of these two indexes, the sum of both being known.

For $L_h$ heterozygous loci and for a value of the heterozygous index *H,* there are $2^{L-L_h}$ classes, and in each class $2^{L_h}$ distinct ranked genotypes ($2^{L_h-1}$ different genotypes). Knowing that there are $C_L^{L-L_h}$ ways to obtain a heterozygous signature of this length, there are therefore $C_L^{L-L_h} 2^{L-L_h}$ families for $L_h$ heterozygous loci. Therefore, altogether there are:

$$\sum_{L_h=0}^{L} C_L^{L_h} 2^{L-L_h} = \sum_{L_h=0}^{L} \frac{L!}{L_h!(L-L_h)!} 2^{L-L_h} = (1+2)^L = 3^L$$

recombination classes. This number of classes correspond to three distinct states $e_l = 0,1,2$ for each locus, which leads to number $3^L$ for locus *L*. This means that all the genotypes of a same class are identical if the phase of the heterozygous loci is not considered. The three possible states permit that a class can be targeted with a base 3 coding; if we define a basic vector:

$$[a_l]_L = 3^{L-l}$$

It is then possible to compute a unique locating index c of the class:

$$c = {}_L\langle a \| e \rangle + 1$$

If the operator providing index **c** of the class is designated by $\hat{C}$:

$$c = \hat{C}\ (i,j),$$

the filtering function can be rewritten as:

$$f_{uv/ij} = F\left\{\hat{C}(u,v) - \hat{C}(i,j)\right\}$$

II.D.1.c. Computing Recombination Probabilities

As set forth hereinabove, $\sigma_l^c$ value, indicating or not an allele change at locus **l**, can indifferently be **0** or **1** if this locus is homozygous. The recombination probability computation can therefore carry out the summation of the two possibilities $\sigma_l^c = \mathbf{0}$ and $\sigma_l^c = 1$ at each homozygous locus. For $L_h$ heterozygous loci, there will thus be $2^{L-L_h}$ terms to sum, which can represent an important use of computation time. Another way to take into account the degeneration introduced by the presence of homozygous loci is to reduce the genotype to heterozygous loci only, compute the equivalent recombination coefficients, and then compute the recombination probability. The summation occurs therefore in an implicit way. The reduction of the genotype occurs very easily: having vector $|d$ for the cumulated distances at each locus, the reduction consists in suppressing components $d_l$ corresponding to the homozygous loci.

The values of the coefficients or recombination can then be obtained by inverting Haldane's map function:

$$r_l = \frac{1}{2}\left\{1 - exp\left[-2(d_{l+1} - d_l)\right]\right\}$$

The probability computation is then carried out by using the expression set forth hereinabove.

II.D.2. Representative Explanation for the Probabilities Associated with Progeny

This section introduces the expression of probabilities $P_{uv/ij}$ for recombination **(i,j)** → **(u,v)** in the general expression of progeny probabilities ${}_t P_{uv}$ established in section II. Given:

$$P_{um/ij} = Pr\left\{ {}_{t-1}^{w}\left[\tilde{G}\right]_{um} \,\Big|\, {}_{t-1}^{w}\left[G\right]_{ij} \right\}$$

$$ {}_{t-1}^{w(3-w)}P_{iji'j'} = Pr\left\{ {}_{t-1}^{w}\left[G\right]_{ij} \,\&\, {}_{t-1}^{3-w}\left[G\right]_{i'j'} \right\},$$

the general expression of the solution can be written:

$$_t P_{uv} = \frac{1}{2} \sum_{w=1}^{2} \sum_{m=1}^{N} \sum_{n=1}^{N} \sum_{i=1}^{N} \sum_{j=1}^{N} \sum_{i'=1}^{N} \sum_{j'=1}^{N} P_{um/ij} \; P_{vn/i'j'} \; {}^{w(3-w)}_{t-1} P_{iji'j'}$$

The probabilities of transfer as product can be written:

$$P_{um/ij} = f_{um/ij} \; \tilde{P}_{um/ij}$$

$$P_{vn/i'j'} = f_{vn/i'j'} \; \tilde{P}_{vn/i'j'} \;,$$

where $f_{um/ij}$ is the filtration function defined hereinabove. By injecting them in the general expression, a more compact expression is obtained. To illustrate the mechanism for reducing summation by the constraints contained in the filtration function, instance probability $P_{um/ij}$:

$$P_{um/ij} = F\left\{H(u,m) - H(i,j)\right\} F\left\{i+j-u-m\right\} \tilde{P}_{um/ij}$$

The summation on index $m$ can be eliminated by imposing identity $m = i + j - u$; the summation on this index is written as:

$$\sum_{m=1}^{N} P_{um/ij} = \sum_{m=1}^{N} \left\{ F\left\{H(u,m) - H(i,j)\right\} F\left\{i+j-u-m\right\} \tilde{P}_{um/ij} \right\},$$

which yields:

$$\sum_{m=1}^{N} P_{um/ij} = F\left\{H(u, i+j-u) - H(i,j)\right\} F\left\{i+j>u\right\} \tilde{P}_{u(i+j-u)/ij}$$

The factor $F\{i + j > u\}$ has the following meaning: by extending function $F$ definition, this factor is non null and equal to a unit if and only if condition $i + j > u$ is verified. This factor comes from the constraint $m > 0$ imposed implicitly by a summation on this index beginning with the unit value. This constraint is contained in the constraint imposing an identical heterozygous signature. Given $|h$, the heterozygous signature vector common to both couples, the indexes are deduced from the operations:

$$i = {}_L\left\langle b \middle| g_i \right\rangle + 1$$

$$j = {}_L\left\langle b \middle| g_j \right\rangle + 1$$

$$u = {}_L\left\langle b \middle| g_u \right\rangle + 1$$

Therefore, the sum of the first two indexes can be expressed as:

$$i+j= {}_L\langle b|g_i+g_j\rangle +2= {}_L\langle b\|h\rangle.|g_i+g_j\rangle +2 {}_L\langle b\|\bar{h}\rangle.|g_i\rangle +2$$

And the third one can be written:

$$u= {}_L\langle b\|h\rangle.|g_u\rangle + {}_L\langle b\|\bar{h}\rangle.|g_u\rangle +1$$

Finally, by subtracting:

$$i+j-u= {}_L\langle b\|h\rangle.|g_i+g_j-g_u\rangle + {}_L\langle b\|\bar{h}\rangle.|g_i\rangle +1$$

Now the minimum value of this difference intervenes in the configuration where all homozygous loci value is null; and where at the same time all the heterozygous loci of the final genotype are such that the upper allele is equal to unity. This configuration can be translated by the two identities:

$$ {}_L\langle b\|\bar{h}\rangle.|g_i\rangle =0$$

$$ {}_L\langle b\|h\rangle.|g_i+g_j-g_u\rangle =0$$

In this extreme situation, the sum of the indexes is equal to:

$$i+j-u=1$$

The condition *i + j > u* is therefore included in the condition *H(u,i +j-) = H(i,j);* its explicit expression constitutes therefore a redundancy that can be eliminated. The summation on index m is limited to:

$$\sum_{m=1}^{N} P_{um/ij} = F\left\{ H(u,i+j-u) - H(i,j)\right\} \tilde{P}_{u(i+j-u)/ij}$$

,

resulting in the expression:

$$ {}_t P_{uv} = \frac{1}{2}\sum_{w=1}^{2}\ \sum_{i=1}^{N}\sum_{j=1}^{N}\sum_{i'=1}^{N}\sum_{j'=1}^{N} F\left\{ H(u,i+j-u)-H(i,j)\right\} \tilde{P}_{u(i+j-u)/ij}$$
$$F\left\{ H(v,i'+j'-v)-H(i',j')\right\} \tilde{P}_{v(i'+j'-v)/i'j'}\ {}^{w(3-w)}_{t-1} P_{iji'j'}$$

II.D.3. Application to Both Fertilization Configurations

To customize the expression of the result established for each problem configuration, two types of configurations can be distinguished:

- cross-fertilization
- self-fertilization

II.D.3.a. Cross-fertilization

The independence of the parents allows the probability of their co-occurrence to be written as the product of their occurrences:

$$Pr\left\{ {}_{t-1}^{w}[G]_{ij} \ \& \ {}_{t-1}^{3-w}[G]_{i'j'} \right\} = Pr\left\{ {}_{t-1}^{w}[G]_{ij} \right\} Pr\left\{ {}_{t-1}^{3-w}[G]_{i'j'} \right\}$$

Therefore:

$$ {}_{t-1}^{w(3-w)}p_{iji'j'} = {}_{t-1}^{w}p_{ij} \ {}_{t-1}^{3-w}p_{i'j'} $$

Consequently, the general expression is written as:

$$ {}_{t}p_{uv} = \frac{1}{2}\sum_{w=1}^{2} \ \sum_{i=1}^{N}\sum_{j=1}^{N}\sum_{i'=1}^{N}\sum_{j'=1}^{N} F\left\{H(u, i+j-u) - H(i,j)\right\} \tilde{P}_{u(i+j-u)/ij} \ {}_{t-1}^{w}p_{ij} $$
$$ F\left\{H(v, i'+j'-v) - H(i',j')\right\} \tilde{P}_{v(i'+j'-v)/i'j'} \ {}_{t-1}^{3-w}p_{i'j'} $$

It can be arranged under the factorized form provided below, where each factor represents the probability to generate a gamete provided by a given parent:

$$ {}_{t}p_{uv} = \frac{1}{2}\sum_{w=1}^{2}\left\{ \left[\sum_{i=1}^{N}\sum_{j=1}^{N} F\left\{H(u, i+j-u) - H(i,j)\right\} \tilde{P}_{u(i+j-u)/ij} \ {}_{t-1}^{w}p_{ij}\right] \left[\sum_{i'=1}^{N}\sum_{j'=1}^{N} F\left\{H(v, i'+j'-v) - H(i',j')\right\} \tilde{P}_{v(i'+j'-v)/i'j'} \ {}_{t-1}^{3-w}p_{i'j'}\right] \right\} $$

II.D.3.b. Self-fertilization

From the expression:

$$ Pr\left\{ {}_{t-1}^{w}[G]_{ij} \ \& \ {}_{t-1}^{3-w}[G]_{i'j'} \right\} = Pr\left\{ {}_{t-1}^{w}[G]_{ij} \ \middle| \ {}_{t-1}^{3-w}[G]_{i'j'} \right\} Pr\left\{ {}_{t-1}^{3-w}[G]_{i'j'} \right\} $$

and describing the identity of the parents by:

$$Pr\left\{{}_{t-1}^{w}[G]_{ij}\Big|{}_{t-1}^{3-w}[G]_{i'j'}\right\}=F\left\{i-i'\right\}F\left\{j-j'\right\}$$

the following equation results:

$${}^{w(3-w)}_{t-1}P_{iji'j'}=F\left\{i-i'\right\}F\left\{j-j'\right\}{}_{t-1}^{w}P_{ij}$$

In addition, the parents' property of identity is verified:

$${}^{w}p_{ij}={}^{3-w}p_{ij}$$

By injecting this result in the general expression of the result, the following equation is obtained:

$$_{t}P_{uv}=\sum_{i=1}^{N}\sum_{j=1}^{N}F\left\{H(u,i+j-u)-H(i,j)\right\}\tilde{P}_{u(i+j-u)/ij}$$
$$F\left\{H(v,i+j-v)-H(i,j)\right\}\tilde{P}_{v(i+j-v)/ij}\;{}_{t-1}P_{ij}$$

In the specific situation of a $F_1$ hybrid self-fertilization, the following properties are verified: first, the parents 1 and 2 are identical; second, a distinct transition corresponds to each recombination state. This brings forth the following properties:

$$i_0=N\;,\;j_0=1\;,\;K=1$$

$$p_{ij}=F\left\{i-i_0\right\}F\left\{j-j_0\right\}$$

By injecting these properties in the general expression of the result, it is determined that:

$$_{t}P_{uv}=\pi_{s_{u(N+1-u)/N}}\pi_{s_{v(N+1-v)/N}}$$

III. Methods for Calculating a Genetic Value Distribution

The presently disclosed subject matter also provides methods for calculating a genetic value distribution. In some embodiments, the presently disclosed subject matter provides methods for calculating a genetic value distribution. In some embodiments, the methods comprise (a) providing a first breeding partner and a second breeding partner, wherein (i) the genotype of each of the first breeding partner and the second breeding partner is known or is predictable with respect to one or more genetic markers linked to one or more genetic loci; (ii) a genetic distance between each genetic marker and the genetic locus to which it is linked is known or can be assigned; and (iii) each genotype is associated with a genetic value; (b) calculating, simulating, or combinations of calculating and simulating a breeding of the first breeding partner and the second breeding partner to generate a subsequent generation, each member of the subsequent generation comprising a genotype; and (c) calculating a genetic value distribution for one or more of the genotypes.

As used herein, the phrase "genetic value" refers to a value assigned to a particular allele at a locus. Alternatively, the phrase "genetic value" can refer to a value assigned to a genotype and/or haplotype. In some embodiments, the genetic value of a genotype and/or a haplotype is calculated by adding together one or more of the individual genetic values that have been assigned for those alleles that make up the genotype and/or the haplotype.

In some embodiments, the genetic values for each allele at each locus is assigned a value of -1 if the allele is desirable in the progeny, a value of -1 if the allele is undesirable in the progeny, and a value of 0 if the allele is neither desirable nor undesirable in the progeny. In these embodiments, the total genetic value that each individual might have at a given genetic locus will be selected from among -2, -1, 0, 1, and 2.

In some embodiments of the presently disclosed subject matter, a genetic value for an allele at each locus is assigned based on a qualitative assessment of the desirability of a given allele being present in a progeny individual. In these embodiments, a genetic value can have any value (e.g., a positive value, a negative value, or zero) including whole numbers, fractional values, decimal values (e.g., numbers with 1, 2, 3, 4, or more decimal places), etc. These values can be assigned in any manner, and can, for example, take into account a degree of contribution that an allele has on the expression of a quantitative trait. In some embodiments, the degree of contribution is determined experimentally by examination of individuals with known genotypes.

A plant is represented by a set of genotypes, each one being affected by an occurrence probability measurement. Until now, a genotype including all marker loci and QTLs was considered. This genotype was noted as $G$ and a particular state of it as $G_{ij}$. Henceforth, each type of locus will be distinguished. The set of marker loci can be noted $E$ while that of QTLs will be noted $U$.

Given $(\xi)$ the name given to a specific plant, given $p_{ij}^{(\xi)}$ the probability of occurrence associated to genotype $G_{ij}$. The expression of probabilities can be denoted $p_{ij}^{(\xi)}$. In order to avoid index multiplication, it can be assumed that the experimental plant $(\xi)$ comes from the generic plant of order $(0)$; so, the probabilities $p_{ij}^{(\xi)}$ are deducted from probabilities $p_{ij}$ characterizing the generic plant. Given that $p_{ij} = Pr\{G_{ij}\}$, it is also true that:

$$p_{ij}^{(\xi)} = Pr\left\{G_{ij} \mid E^{(\xi)}\right\}.$$

This relation indicates that the marking introduces a condition of conformity from the global genotype to the genotype measured at the marker locus. To establish the expression of this conditional probability, Bayes' theorem can be employed:

$$p_{ij}^{(\xi)} = Pr\left\{G_{ij} \mid E^{(\xi)}\right\} = \frac{Pr\left\{E^{(\xi)} \mid G_{ij}\right\} Pr\left\{G_{ij}\right\}}{\sum_{i=1}^{N}\sum_{j=u}^{N} Pr\left\{E^{(\xi)} \mid G_{ij}\right\} Pr\left\{G_{ij}\right\}}$$

The probability under evaluation can be completely determined when the conditional probability $Pr\{E^{(\xi)}|G_{ij}\}$ is actualized. This probability will be null if $E^{(\xi)} \neq E_{uv}$ and will be equal to unity on the contrary. Using once more function $F$, this probability can be written as:

$$Pr\left\{E^{(\xi)}\,|\,G_{ij}\right\}=F\left\{E_{ij}-E^{(\xi)}\right\}$$

In this way, one arrives at the expression:

$$p_{ij}^{(\xi)}=\frac{F\left\{E_{ij}-E^{(\xi)}\right\}p_{ij}}{\sum_{i=1}^{N}\sum_{v=j}^{N}F\left\{E_{ij}-E^{(\xi)}\right\}p_{ij}}$$

III.A. Computation of the Progeny Index Distribution

In some embodiments, an index distribution associated with plant crossings and/or self-fertilizations can be computed as set forth hereinbelow.

III.B. Definition of an Additive Index

A simple index $I^{(\gamma)}$ can be defined wherein a subset of QTLs intervenes among the set of the K QTLs, by calculating the following weighted sum:

$$I^{(\gamma)}=\sum_{v=1}^{K}\alpha_{v}^{(\gamma)}Q_{v}$$

This can also be written under the scalar product form:

$$I^{(\gamma)}=\left\langle\alpha^{(\gamma)}\,|\,Q\right\rangle$$

where vector $|Q$ is the vector of the state of all the QTLs, of element $Q_v$, defined by:

$Q_v = 1$ for the configuration: $AA \Leftrightarrow (1,1)$
$Q_v = 0$ for the configuration: $Aa \Leftrightarrow (1,0)$
$Q_v = 0$ for the configuration: $aA \Leftrightarrow (0,1)$
$Q_v = -1$ for the configuration: $aa \Leftrightarrow (0,0)$

Taking into account this definition of element $Q_v$, the index value can be independent of the phase at the loci. Therefore, all the genotypes of a same class will have the same index values. Consequently, a maximum of $3^{K(\gamma)}$ distinct index values can exist if $K^{(\gamma)}$ is the number of QTLs intervening in the evaluation of index $I^{(\gamma)}$.

Vector $|\alpha^{(\gamma)}$ is defined as follow: If $\left|S_q^{(\gamma)}\right\rangle$ is defined as the position signature vector of $K$ QTLs intervening in the index computation of which the components are such that:

$[S_q]_v = 1$ if the locus with coefficient **v** contributes to index (y) value
$[S_q]_v = 0$ if the locus with coefficient **v** doesn't contribute to index (y) value

the vector of coefficients $|\alpha^{(\gamma)}$, length **K,** and element can be defined:

$$\alpha_v^{(\gamma)} \neq 0 \quad \text{if the locus with coefficient } \textbf{\textit{v}} \text{ contributes to index (y) value}$$

$$\alpha_v^{(\gamma)} = 0 \quad \text{if the locus with coefficient } \textbf{\textit{v}} \text{ doesn't contribute to index (y) value}$$

Complex indexes made of combined simple indexes can also be defined:

$$J = \sum_{\gamma=1}^{n_{ind}} \beta_\gamma I^{(\gamma)} = \sum_{v=1}^{K} \left[ \sum_{\gamma=1}^{n_{ind}} \beta_\gamma \alpha_v^{(\gamma)} \right] Q_v$$

This can also be written as:

$$J = \left\langle w \mid Q \right\rangle$$

Where vector $|w$ of component: $w_v = \sum_\gamma \beta_\gamma \alpha_v^{(\gamma)}$ has been defined. If matrix $\hat{\alpha}$ of dimensions (**K**, $\textbf{\textit{n}}_{ind}$) and element

$\alpha_{v\gamma} = \alpha_v^{(\gamma)}$ is defined, the vector of coefficients $\textbf{\textit{w}}_v$ can be expressed as:

$$\left| w \right\rangle = \hat{\alpha} \left| \beta \right\rangle$$

The calculation of a complex index is therefore the same as that of a simple index when taking into account the computation of the adapted coefficients.

An additivity property for such indexes can be shown. For this purpose the index definition can be rewritten by revisiting the genotypic representation by a unique vector. Hence, given $|e_{uv}$, the vector associated with genotype **(u, v)**; given $|e^{(q)}_{uv}$, the QTLs genotype, and $\|e^{(q)}\|_{uv}$, the associated experimental genotype. Taking into consideration the index definition and the nature of coding by a single vector, the index value can be expressed as:

$$I_{uv} = \left\langle w \left\| e^{(q)} \right| - 1 \right\rangle_{uv}$$

If on the other hand, the relation:

$$\left\| e^{(q)} \right\rangle_{uv} = \left| \breve{g}^{(q)} \right\rangle_{uv} + \left| \widehat{g}^{(q)} \right\rangle_{uv}$$

is recalled, the index can be rewritten as follows:

$$I_{uv} = \left\langle w \middle| \breve{g}^{(q)} - \frac{1}{2} \right\rangle_u + \left\langle w \middle| \widehat{g}^{(q)} - \frac{1}{2} \right\rangle_v$$

Therefore, each haplotype can be expressed as carrying an index value, which can be defined by:

$$I_u = \left\langle w \middle| g^{(q)} - \frac{1}{2} \right\rangle_u$$

And the sum of these values yields the index value of the genotype:

$$I_{uv} = I_u + I_v$$

This additivity property can be used advantageously and widely to select plants according to the index distribution of their gametes, and therefore avoid progeny simulations that have little value in terms of the distribution of the index value sums.

III.C. Other Index Definitions

Many other definitions can be considered without the additivity property because of determinations based on non-linear functions. In particular, the configurations of dominance where "maximum" functions occur can be considered. The additivity property is not exploited by the evaluation method of its distribution for the index computation to keep its general character. It is evaluated from the complete plant, thus independently from the relationship between an index value and the index values of the gametes involved.

III.D. Expressing the Index Distribution

Above, for the sake of highlighting the index additivity property, an index value was isolated through haplotype indexes. Now, for $K^{(\gamma)}$ QTLs involved in establishing a specific index $I^{(\gamma)}$, there will be at most $3^{K(\gamma)}$ distinct index values: *i.e.,* at most up to the number of recombination classes. Therefore, criteria distribution $I^{(\gamma)}$ associated to the experimental plant ($\xi$) can be expressed as:

$$Pr\left\{ I^{(\gamma)} \middle| \xi \right\} = \sum_{c=1}^{N_{ind}^{(\gamma)}} q_c^{(\xi)} F\left\{ I^{(\gamma)} - I_c^{(\gamma)} \right\}$$

where $N_{ind}^{(\gamma)} = 3^{K^{(\gamma)}}$ is the number of index values, where $K^{(y)}$ is the number of QTLs intervening in index $I^{(y)}$ computation, where $I_c^{(y)}$ is the value of the index associated with the class c coefficient, and where $q_c$ is the associated probability.

In order to obtain probability $q_c$, two steps can be considered. The first one involves summing over all genotypic states not taking part in the specific index calculation. This summation presents an interest because it reduces to $4^{K(\gamma)}$ the number of ranked states for the value $4^L$. It is realized by conserving the initial size of the genotype, but by arbitrarily

attributing homozygous state $e_l = 0$ to the genotypes not involved (non involved markers and QTLs). If one designates by $\widehat{\Sigma}$ and $\breve{\Sigma}$ the operators realizing the computation actions of the new indexes:

$$i = \widehat{\Sigma}\left|e\right\rangle_{kl},$$

the summation operation can be written:

$$j = \breve{\Sigma}\left|e\right\rangle_{kl}$$

with $N = 2^L$.

The second step aims at generating a population integrated with the phase states. By definition, this comes to compound individuals belonging to a same class: with $(k_1, l_1), (k_2, l_2), ..., (k_{m_c}, l_{m_c})$ as individual indexes of a class c including $m_c$ of them; the indexes of the various genotypes of the class are then such as:

$$c = \hat{C}\left(k_1, l_1\right) = \hat{C}\left(k_2, l_3\right) = ... = \hat{C}\left(k_{m_c}, l_{m_c}\right)$$

Consequently, the coding of the summation is:

$$q_c^{(\xi)} = \sum_{i=1}^{N}\sum_{j=1}^{N}\lambda_{ij}^{(\xi)}F\left\{c - \hat{C}(i,j)\right\}$$

So, the index distribution calculation from probabilities $p_{kl}^{(\xi)}$ entails the operations:

$$\lambda_{ij}^{(\xi)} = \sum_{k=1}^{N}\sum_{l=1}^{N}p_{kl}^{(\xi)}F\left\{i - \widehat{\Sigma}\left|e\right\rangle_{kl}\right\}F\left\{j - \breve{\Sigma}\left|e\right\rangle_{kl}\right\}$$

$$q_c^{(\xi)} = \sum_{i=1}^{N}\sum_{j=1}^{N}\lambda_{ij}^{(\xi)}F\left\{c - \hat{C}(i,j)\right\}$$

$$Pr\left\{I^{(\gamma)}\middle|\xi\right\} = \sum_{c=1}^{N_{ind}^{(\gamma)}}q_c^{(\xi)}F\left\{I^{(\gamma)} - I_c^{(\gamma)}\right\}$$

IV. Methods for Choosing a Breeding Pair for Producing a Progeny Having a Desired Genotype

The presently disclosed subject matter also provides methods for choosing a breeding pair for producing a progeny having a desired genotype. In some embodiments, the methods comprise (a) providing a first breeding partner and a second breeding partner, wherein (i) the genotype of each of the first breeding partner and the second breeding partner is known or is predictable with respect to one or more genetic markers, each of which is linked to a genetic locus; and (ii) a genetic distance between each genetic marker and the genetic locus to which it is linked is known or can be assigned; (b) calculating, simulating, or combinations of calculating and simulating a breeding of the first breeding partner and the second breeding partner to generate a subsequent generation, each member of the subsequent generation comprising a genotype; (c) calculating a distribution of a probability or a frequency of occurrence for one or more of the genotypes of one or more members of the subsequent generation; (d) repeating steps (a) through (c) with a different first, different second, or both different first and different second potential breeding partners; (e) comparing the probability or frequency distributions calculated in one or more iterations of step (c) to each other; and (f) choosing a breeding pair based on the comparing step.

In some embodiments, the presently disclosed methods for choosing a breeding pair for producing a progeny having a desired genotype comprise (a) providing a first breeding partner and a second breeding partner, wherein (i) the genotype of each of the first breeding partner and the second breeding partner is known or is predictable with respect to one or more genetic markers linked to one or more genetic loci; (ii) a genetic distance between each genetic marker and the genetic locus to which it is linked is known or can be assigned; and (iii) each genotype is associated with a genetic value; (b) calculating, simulating, or combinations of calculating and simulating a breeding of the first breeding partner and the second breeding partner to generate a subsequent generation, each member of the subsequent generation comprising a genotype; (c) calculating a distribution of genetic values associated with one or more of the genotypes of one or more members of the subsequent generation; (d) repeating steps (a) through (c) with a different first, different second, or both different first and different second potential breeding partners; (e) comparing the genetic value distributions calculated in one or more iterations of step (c) to each other; and (f) choosing a breeding pair based on the comparing step.

Additionally, in some embodiments the presently disclosed methods further comprise generating one or more further generation progeny, wherein each further generation progeny is generated by one or more rounds of calculating, simulating, or combinations of calculating and simulating a breeding of at least one member of the subsequent generation or a later generation with an individual selected from the group consisting of itself, a member of the immediately prior generation, another individual from the same generation, another individual from a previous generation, the first breeding partner, the second breeding partner, and doubled haploid derivatives thereof. Distributions of probabilities and/or frequencies of occurrence for one or more of the genotypes of one or more members of any such further generation, and/or distributions of genetic values associated with one or more of the genotypes of one or more members of any of the further generations can also bee calculated and compared.

Thus, the presently disclosed methods in some embodiments allow for the selection of breeding pairs based on a comparison of the distributions of the probability or frequency of occurrence of one or more of the genotypes and/or of the distributions of the genetic values associated with these genotypes in the subsequent generation and/or of any further generation. The choice of a breeding pair based on comparing one or more of these distributions can include any criteria deemed relevant, and can include, but is not limited to the number of generations required to produce an individual with a genotype having a desired minimum genetic value, the extent to which genetic values can be increased by increasing the number of generations, and judgments that take into account both probabilities and/or frequencies of generating desirable genotypes in conjunction with the genetic values of the desirable genotypes. It is understood that the presently disclosed subject matter is not limited to any single criterion in the comparing step leading to the choice of breeding partners.

In some embodiments, an exemplary approach to selecting breeding pairs is to stochastically simulate progeny frequency or index distributions through the simulation of meiosis (the creation of gametes) and fertilization (the union of gametes). Meiosis can be seen as a series of recombination events along a given chromosome happening either at random or not while homologous chromosomes separate into gametic sets. Progeny genotype, GEN, then results from the union of two gametic sets of chromosomes, respectively with genotypes GEH1 and GEH2, through fertilization.

Because each series of recombination events can give rise to different gametes displaying different allelic configurations, there are many possible progeny genotypes, each with an associated frequency or probability of occurrence. All progeny genotypes, with their associated frequency or probability of occurrence, can be represented by a frequency or probability distribution.

By way of example, representative genotypes can be diploid, with two alleles, "a" and "A". In some embodiments, alleles can also be coded numerically using a = 0 and A = 1.

In this example, there are up to four possible "phased" genotypes (GEN) at each locus for an individual: aa, aA, Aa, and AA, where the first letter in the genotype refers to the allele contributed by the first breeding partner of the breeding that resulted in the individual (GEH1), and the second letter refers to the allele contributed by the second breeding partner of the same breeding (GEH2). "Phased" genotypes are genotypes that take into account the parental origin of the alleles. "Unphased" genotypes do not take into account the parental origin of the alleles. As such, at each locus there are up to three possible "unphased" genotypes: aa, aA (which is equivalent to Aa), and AA. Because there can be more phased than unphased genotypes at a given locus, several phased genotypes can correspond to an unphased genotype (heterozygous loci). When considering several loci, one individual can be represented by more than one phased, multi-locus, genotype, each genotype being referred to as a sample genotype.

Phased genotypes can be coded numerically using, for example, aa = 0, Aa =1, aA = 2, and AA = 3.

Unphased genotypes can be coded numerically using, for example, aa = 0, aA = 1, and AA = 2.

It can be seen that numerical codes for phased genotypes follow the rule:

$$GEN = GEH1 + 2 \times GEH2$$

Experimental genotypes are in some embodiments unphased. In order to simulate progeny genotypes, it can first be necessary to simulate frequency distributions of phased genotypes underlying unphased (experimental) genotypes. This can be achieved in some embodiments by simulating meiosis and fertilization of individuals.

*Generating phased genotypes compatible with experimental genotypes.* By way of an additional example, there can be in some embodiments ns12 sample genotypes for any individual. Sample genotypes for the first breeding partner can be stored in a vector pa1 of length N (N being the size of a linkage group, in terms of number of marker loci) x ns12 (number of sample genotypes). Each pa1 vector can be a series of ns12 subgroups of N values, stored one after another, each subgroup containing values for one sample genotype. Sample genotypes for the second breeding partner can also be stored in a vector pa1, having the same attributes as that of the first breeding partner.

In some embodiments, simulating meiosis can comprise simulating recombination (*i.e.,* crossing-overs) between homologous chromosomes. Recombination can be viewed as "walking" on homologous chromosomes and "jumping" from one to the other or vice-versa. In some embodiments, homologous chromosomes can be defined as one being on the "top" and the other on the "bottom". Indicator variables sw1 and sw2 can be defined to indicate "walking" on either the "top" or the "bottom" chromosome. In some embodiments, these indicator variables can take the following values:

- 1 if "walking" on the "top" chromosome
- 2 if "walking" on the "bottom" chromosome
- sw1 is the indicator variable for the first breeding partner and sw2 the indicator variable for the second breeding partner.

In some embodiments, the first step in simulating meiosis is to pick a random sample genotype from among the ns12 samples for the first breeding partner. To do so, a random number (e.g., "iran") can be generated using, for example, a normalized uniform distribution. The sample genotype at position iran in the vector pa1 can then be picked. The same procedure can be applied to pick a sample genotype for the second breeding partner.

In some embodiments, initial conditions for the simulation can be set as starting at marker locus nn = 1 and on the "top" chromosome (sw1 = 1, sw2 = 1).

A "test" recombination distance $r_j^*$ can be sampled from a normalized uniform distribution. If this test recombination distance is smaller than the known recombination distance, $rn_j$, between marker loci nn and nn + 1 (here $r_j^* < r1_j$, where $r1_j$ is the known recombination distance between marker locus 1 and marker locus 2), the value of indicator variable sw1 changes from 1 to 2 (or 2 to 1 - here from 1 to 2). Genetically, this indicates that a recombination has taken place between marker loci nn and nn + 1 (here 1 and 2), "jumping" from one to the other homologous chromosome (here the "top" to the "bottom" chromosome). If the "test" recombination distance is larger than the known recombination distance, $rn_j$, the indicator variable sw1 remains unchanged. Genetically, this indicates that no recombination has taken place between marker loci nn and nn + 1 (here 1 and 2), "walking" continuously on the same homologous chromosome (here the "top" chromosome). The same steps can be carried out for the second breeding partner.

Gametes created from the first breeding partner, with genotype GEH1, can be derived through the following steps (the same steps can apply to the creation of gametes from the second breeding partner with genotype GEH2):

- if the first breeding partner sample genotype is homozygous at the marker locus, the value of sw1 can be considered irrelevant because "top" and "bottom" alleles are the same. If the genotype of the first breeding partner at this marker locus is of type "aa", then GEH1 = 0. If the genotype of the first breeding partner at this marker locus is of type "AA", then GEH1 = 1.
- if the first breeding partner sample genotype is heterozygous at the marker locus, the value of sw1 determines GEH1. If the "top" allele at this marker locus is of type "a" and the "bottom" allele of type "A", and sw1 = 1, then GEH1 = 0. If sw1 = 2, then GEH1 = 1. If the "top" allele at this marker locus is of type "A" and the "bottom" allele of type "a", and sw1 =1, then GEH1 =1. If sw1 = 2, then GEH1 = 0.

Once gametes from the first and the second breeding partner have been created, with genotypes GEH1 and GEH2, a progeny genotype, GEN, can be defined by:

$$GEN = GEH1 + 2 \times GEH2$$

This sample genotype (phased genotype), at marker locus nn =1 can be compared to the experimental marker genotype (unphased genotype) of an individual. If the sample genotype is compatible with the experimental genotype, the sample genotype is added to an "output" vector, containing a pre-defined target number of sample genotypes. The output vector is of size N x ns (ns being the pre-defined target number of sample genotypes).

Each of the N marker loci can be approached in the same fashion, starting at the step where a "test" recombination distance $r_j^*$ is sampled when moving to the subsequent marker locus. These steps can then be repeated ns times to obtain ns sample genotypes.

If, for an intermediate marker locus nn = k, the sample genotype is incompatible with the experimental marker genotype, then the entire sample genotypes, from nn =1 to nn = k are discarded and the process initiated anew at the very beginning of meiosis simulation: i.e., with picking a random sample genotype from among the ns12 samples for the first breeding partner, and then the second breeding partner.

*Simulating future progeny.* The process to simulate future progeny can be essentially the same, without the comparison between sample genotype and experimental genotype since no experimental genotype is available for future progeny. Also, in some embodiments initial sample genotypes are not chosen randomly but rather the sample genotypes created above are used.

QTL genotypes can be computed from sample genotypes using the matrices proposed by Fisch *et al.,* 1996. Genetic values can then be computed based on QTL genotypes using economic indices such as:

$$GV = \sum_t \beta_t \sum_q \alpha_{qt} \sum_i p_{iqt} \delta_{iqt}$$

where $\beta_t$ is the weight (economic value) of trait t, $\alpha_{qt}$ is the effect of the favorable allele at QTL q of trait t (usually the additive value of the QTL), $p_{iqt}$ is the probability of occurrence of genotype i at QTL q of trait t, and $\delta_{iqt}$ is the selection value of QTL genotype i at QTL q of trait t.

V. Methods for Generating a Progeny Individual Having a Desired Genotype

The presently disclosed subject matter also provides methods for generating a progeny individual having a desired genotype. In some embodiments, the methods comprise (a) providing a first breeding partner and a second breeding partner, wherein (i) the genotype of each of the first breeding partner and the second breeding partner is known or is predictable with respect to one or more genetic markers, each of which is linked to a genetic locus; and (ii) a genetic distance between each genetic marker and the genetic locus to which it is linked is known or can be assigned; (b) calculating, simulating, or combinations of calculating and simulating a breeding of the first breeding partner and the second breeding partner to generate a subsequent generation, each member of the subsequent generation comprising a genotype; (c) calculating a distribution of a probability or a frequency of occurrence for one or more of the genotypes of one or more members of the subsequent generation; (d) repeating steps (a) through (c) with a different first, different second, or both different first and different second potential breeding partners; (e) comparing the probability or frequency distributions calculated in one or more iterations of step (c) to each other; (f) choosing a breeding pair based on the comparing step; and (g) breeding the breeding pair in accordance with the calculating, simulating, or combinations of calculating and simulating as set forth in step (b) to generate a progeny individual having a desired genotype.

In some embodiments, the presently disclosed methods for generating a progeny individual having a desired genotype comprises (a) providing a first breeding partner and a second breeding partner, wherein (i) the genotype of each of the first breeding partner and the second breeding partner is known or is predictable with respect to one or more genetic markers linked to one or more genetic loci; (ii) a genetic distance between each genetic marker and the genetic locus to which it is linked is known or can be assigned; and (iii) each genotype is associated with a genetic value; (b) calculating, simulating, or combinations of calculating and simulating a breeding of the first breeding partner and the second breeding partner to generate a subsequent generation, each member of the subsequent generation comprising a genotype; (c) calculating a distribution of genetic values associated with one or more of the genotypes of one or more members of the subsequent generation; (d) repeating steps (a) through (c) with a different first, different second, or both different first and different second potential breeding partners; (e) comparing the genetic value distributions calculated in one or more iterations of step (c) to each other; (f) choosing a breeding pair based on the comparing step; and (g) breeding the breeding pair in accordance with the calculating, simulating, or combinations of calculating and simulating as set forth in step (b) to generate a progeny individual having a desired genotype.

Accordingly, the presently disclosed methods are designed to produce the desired progeny individual itself by performing the series of breeding steps that were modeled by the methods of the presently disclosed subject matter and that employ the breeding partners through the presently disclosed methods. Thus, the phrase "breeding the breeding pair in accordance with the calculating, simulating, or combinations of calculating and simulating as set forth in step (b)" refers to actually performing the series of breeding steps that the presently disclosed methods indicate would result in producing the desired progeny individual. Since the presently disclosed methods allow for the identification at each breeding stage of the genotypes that should be employed to generate the progeny of the next generation, and one of ordinary skill in the art would understand how to produce each generation and test members of the generation for the desired genotype, one of ordinary skill in the art would be able to perform these breedings and identify appropriate genotypes after consideration of the presently disclosed subject matter.

VI. Methods, Systems, and Computer Program Products

The presently disclosed subject matter also provides methods, systems, and computer program products that can be employed in the general methods disclosed herein.

In some embodiments, the methods of the presently disclosed subject matter can be implemented in hardware, firmware, software, or any combination thereof. In some embodiments, the methods and data structures for calculating a distribution of a probability or a frequency of occurrence of one or more potential genotypes, for calculating a genetic value distribution, for choosing a breeding pair for producing a progeny having a desired genotype, and/or for generating a progeny individual having a desired genotype can be implemented at least in part as computer readable instructions and data structures embodied in a computer-readable medium.

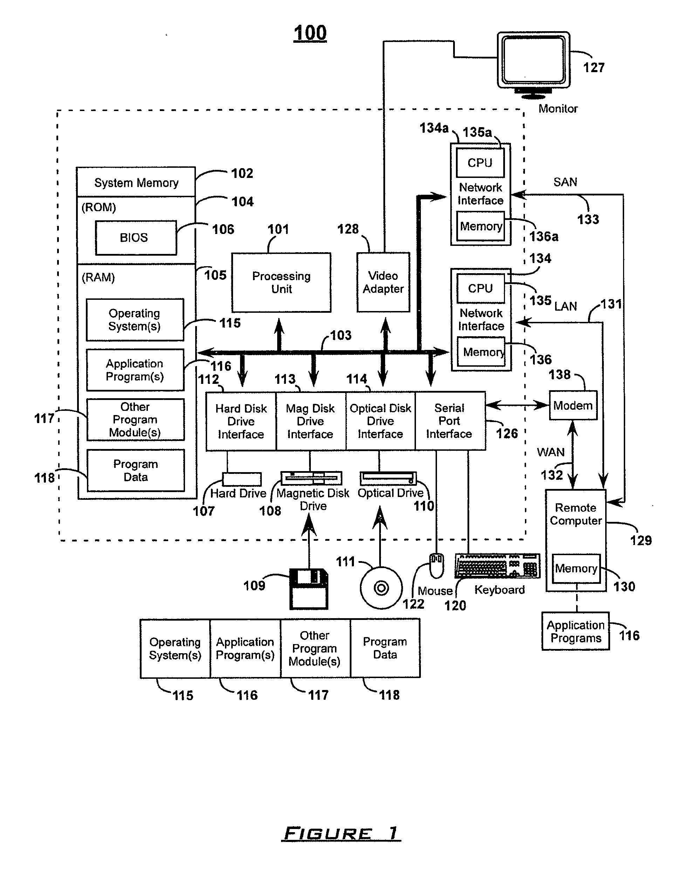

With reference to Figure 1, an exemplary system for implementing the presently disclosed subject matter includes a general purpose computing device in the form of a conventional personal computer **100,** including a processing unit **101,** a system memory **102,** and a system bus **103** that couples various system components including the system memory to the processing unit **101.** System bus **103** can be any of several types of bus structures including a memory bus or memory controller, a peripheral bus, and a local bus using any of a variety of bus architectures. The system memory includes read only memory (ROM) **104** and random access memory (RAM) **105.** A basic input/output system (BIOS) **106,** containing the basic routines that help to transfer information between elements within personal computer **100,** such as during start-up, is stored in ROM **104.** Personal computer **100** further includes a hard disk drive **107** for reading from and writing to a hard disk (not shown), a magnetic disk drive **108** for reading from or writing to a removable magnetic disk **109,** and an optical disk drive **110** for reading from or writing to a removable optical disk **111** such as a CD ROM or other optical media.

Hard disk drive **107,** magnetic disk drive **108,** and optical disk drive **110** are connected to system bus **103** by a hard disk drive interface **112,** a magnetic disk drive interface **113,** and an optical disk drive interface **114,** respectively. The drives and their associated computer-readable media provide nonvolatile storage of computer readable instructions, data structures, program modules, and other data for personal computer **100.** Although the exemplary environment described herein employs a hard disk, a removable magnetic disk **109,** and a removable optical disk **111,** it will be appreciated by those skilled in the art that other types of computer readable media which can store data that is accessible by a computer, such as magnetic cassettes, flash memory cards, digital video disks, Bernoulli cartridges, random access memories, read only memories, and the like may also be used in the exemplary operating environment.

A number of program modules can be stored on the hard disk, magnetic disk **109,** optical disk **111,** ROM **104,** or RAM **105,** including an operating system **115,** one or more applications programs **116,** other program modules **117,** and program data **118.**

A user can enter commands and information into personal computer **100** through input devices such as a keyboard **120** and a pointing device **122.** Other input devices (not shown) can include a microphone, touch panel, joystick, game pad, satellite dish, scanner, or the like. These and other input devices are often connected to processing unit 101 through a serial port interface **126** that is coupled to the system bus, but can be connected by other interfaces, such as a parallel port, game port or a universal serial bus (USB). A monitor **127** or other type of display device is also connected to system bus **103** via an interface, such as a video adapter **128.** In addition to the monitor, personal computers typically include other peripheral output devices, not shown, such as speakers and printers. With regard to the presently disclosed subject matter, the user can use one of the input devices to input data indicating the user's preference between alternatives presented to the user via monitor **127.**

Personal computer **100** can operate in a networked environment using logical connections to one or more remote computers, such as a remote computer **129.** Remote computer **129** can be another personal computer, a server, a router, a network PC, a peer device or other common network node, and typically includes many or all of the elements described above relative to personal computer **100,** although only a memory storage device **130** has been illustrated in Figure 1. The logical connections depicted in Figure 1 include a local area network (LAN) **131,** a wide area network (WAN) **132,** and a system area network (SAN) **133.** Local- and wide-area networking environments are commonplace in offices, enterprise-wide computer networks, intranets and the Internet.

System area networking environments are used to interconnect nodes within a distributed computing system, such as a cluster. For example, in the illustrated embodiment, personal computer **100** can comprise a first node in a cluster and remote computer **129** can comprise a second node in the cluster. In such an environment, it is preferable that personal computer **100** and remote computer **129** be under a common administrative domain. Thus, although computer **129** is labeled "remote", computer **129** can be in close physical proximity to personal computer **100.**

When used in a LAN or SAN networking environment, personal computer **100** is connected to local network **131** or system network **133** through network interface adapters **134** and **134a.** Network interface adapters **134** and **134a** can include processing units **135** and **135a** and one or more memory units **136** and **136a.**

When used in a WAN networking environment, personal computer **100** typically includes a modem **138** or other device for establishing communications over WAN **132.** Modem **138,** which can be internal or external, is connected to system bus **103** via serial port interface **126.** In a networked environment, program modules depicted relative to personal computer **100,** or portions thereof, can be stored in the remote memory storage device. It will be appreciated that the network connections shown are exemplary and other approaches to establishing a communications link between the computers can be used.

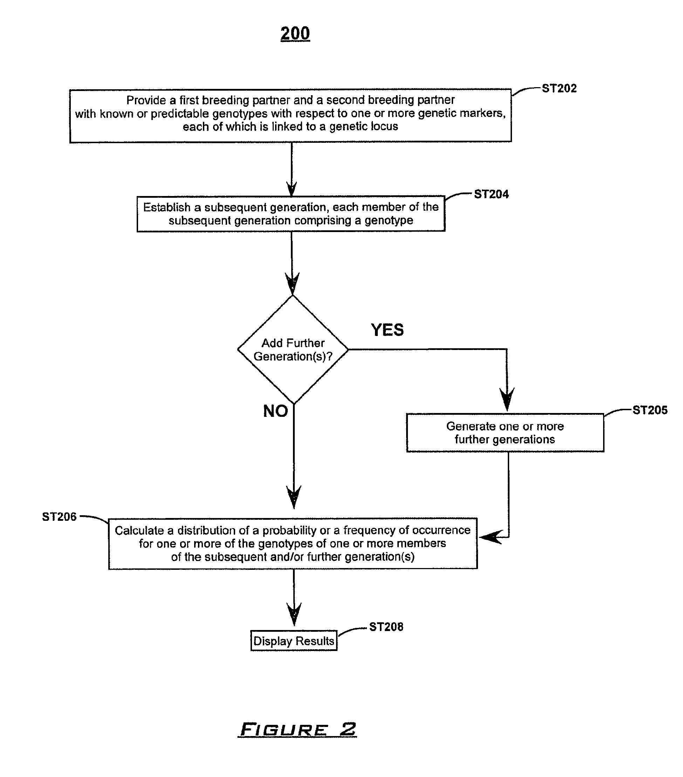

A representative example of an embodiment of the presently disclosed subject matter for calculating a distribution of a probability or a frequency of occurrence of one or more potential genotypes as disclosed herein is referred to generally at **200** in Figure 2.

As shown in step **ST202** in Figure 2, a first breeding partner and a second breeding partner are provided, wherein the genotype of each of the first breeding partner and the second breeding partner is known or is predictable with respect to one or more genetic markers, each of which is linked to a genetic locus. In some embodiments, a genetic distance between each genetic marker and the genetic locus to which it is linked is known.

As shown in step **ST204** in Figure 2, a plurality of subsequent generation genotypes is established by calculating, simulating, or combinations of calculating and simulating a breeding of the first breeding partner and the second breeding partner

If desired, further generations can be generated as shown in step **ST205** in Figure 2, which can be repeated one or more times to generate a plurality of further generation genotypes, each of which is associated with a probability of occurrence or a frequency of occurrence.

As shown in step **ST206** in Figure 2, a distribution of a probability or a frequency of occurrence for each of the plurality of subsequent and/or further generation genotypes is calculated.

As shown in step **ST208** in Figure 2, in some embodiments of the presently disclosed subject matter the results of the calculation in step **ST206** can be displayed. It is noted that this step is optional.

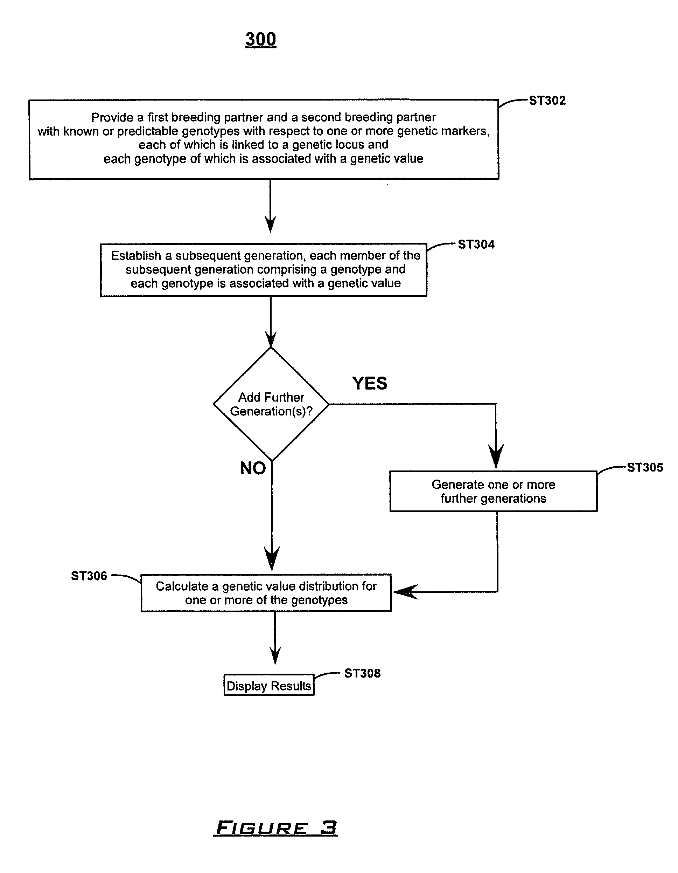

A representative example of an embodiment of the presently disclosed subject matter for calculating a genetic value distribution as disclosed herein is referred to generally at **300** in Figure 3.

As shown in step **ST302** in Figure 3, a first breeding partner and a second breeding partner are provided, wherein the genotype of each of the first breeding partner and the second breeding partner is known or is predictable with respect to one or more genetic markers, each of which is linked to a genetic locus, and each genotype is associated with a genetic value. In some embodiments, a genetic distance between each genetic marker and the genetic locus to which it is linked is known.

As shown in step **ST304** in Figure 3, a plurality of subsequent generation genotypes is established by calculating, simulating, or combinations of calculating and simulating a breeding of the first breeding partner and the second breeding partner.

If desired, further generations can be generated as shown in step **ST305** in Figure 3, which can be repeated one or more times to generate a plurality of further generation genotypes, each of which is associated with a probability of occurrence or a frequency of occurrence.

As shown in step **ST306** in Figure 3, a genetic value distribution of one or more of the subsequent and/or further generation genotypes is calculated. Optionally, in step **ST308** in Figure 3, the results of the calculation in step **ST310** in Figure 3 are displayed.

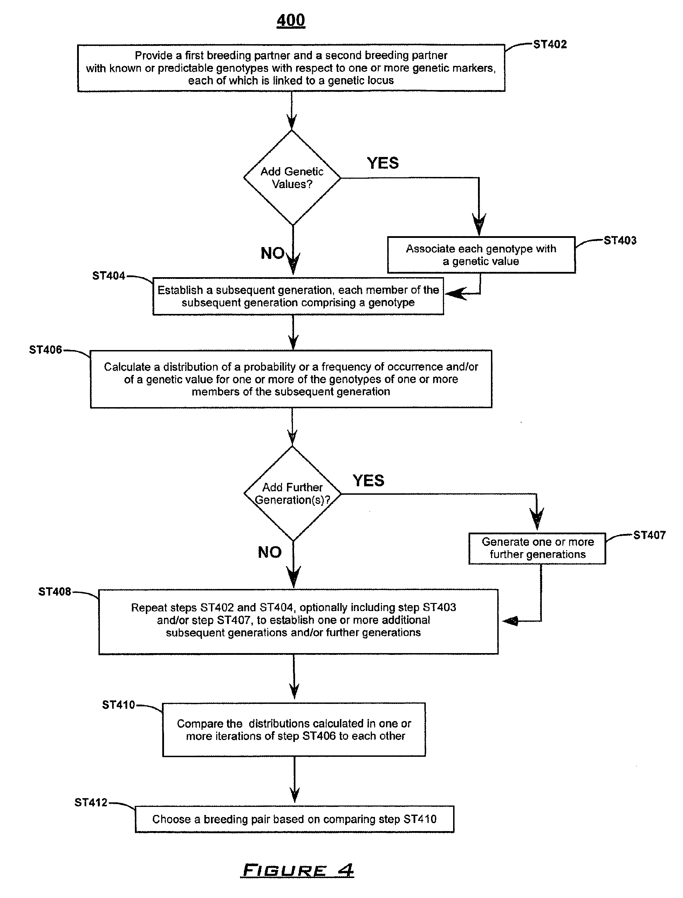

A representative example of an embodiment of the presently disclosed subject matter for producing a progeny having a desired genotype as disclosed herein is referred to generally at **400** in Figure 4.

As shown in step **ST402** in Figure 4, a first breeding partner and a second breeding partner are provided, wherein the genotype of each of the first breeding partner and the second breeding partner is known or is predictable with respect to one or more genetic markers, each of which is linked to a genetic locus. In some embodiments, a genetic distance between each genetic marker and the genetic locus to which it is linked is known.

As shown in step **ST403** in Figure 4, each genotype can be associated with a genetic value, if desired. It is noted that the in addition to associating genetic values to the genotypes of the first and second breeding partners, genetic values can also be associated to any of the genotypes established in the subsequent generation, one or more of the further generations, or combinations thereof.

As shown in step **ST404** in Figure 4, a plurality of subsequent generation genotypes are established by calculating, simulating, or combinations of calculating and simulating a breeding of the first breeding partner and the second breeding partner.

As shown in step **ST406** in Figure 4, a distribution of a probability or a frequency of occurrence and/or of a genetic value for one or more of the plurality of subsequent generation genotypes is calculated.

If desired, further generations can be generated as shown in step **ST407** in Figure 4, which can be repeated one or more times to generate a plurality of further generation genotypes, each of which is associated with a probability of occurrence or a frequency of occurrence and/or with a genetic value.

If desired, one or more of steps **ST402** through **ST407** in Figure 4 can be repeated one or more times in step **ST408** of Figure 4 to generate one or more additional subsequent generations and/or further generations.

As shown in step **ST410** in Figure 4, the distributions calculated in one or more iterations of step **ST406** are compared to each other.

As shown in step **ST412** in Figure 4, a breeding pair is chosen based on comparing step **ST410.**

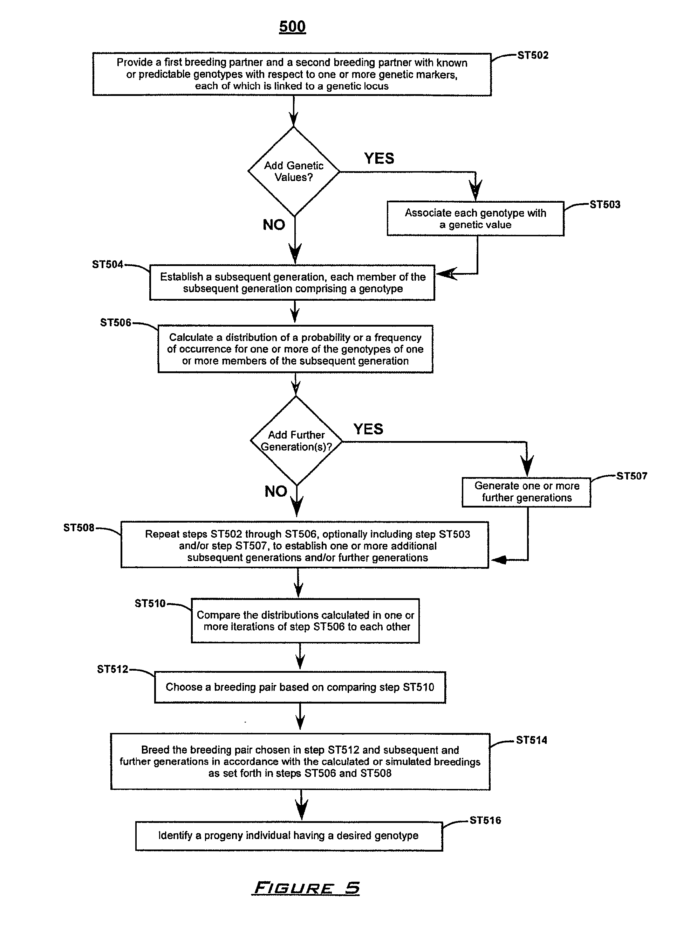

A representative example of an embodiment of the presently disclosed subject matter for generating a progeny individual having a desired genotype as disclosed herein is referred to generally at **500** in Figure 5.

As shown in step **ST502** in Figure 5, a first breeding partner and a second breeding partner is provided, wherein the genotype of each of the first breeding partner and the second breeding partner is known or is predictable with respect to one or more genetic markers, each of which is linked to a genetic locus. In some embodiments, a genetic distance between each genetic marker and the genetic locus to which it is linked is known.

As shown in step ST503 in Figure 5, each genotype can be associated with a genetic value, if desired. It is noted that the in addition to associating genetic values to the genotypes of the first and second breeding partners, genetic values can also be associated to any of the genotypes established in the subsequent generation, one or more of the further generations, or combinations thereof.

As shown in step ST504 in Figure 5, a plurality of subsequent generation genotypes is established by calculating, simulating, or combinations of calculating and simulating a breeding of the first breeding partner and the second breeding partner.

As shown in step ST506 in Figure 5, a distribution of a probability or a frequency of occurrence and/or of a genetic value for one or more of the plurality of subsequent generation genotypes is calculated.

If desired, further generations can be generated as shown in step ST507 in Figure 5, which can be repeated one or more times to generate a plurality of further generation genotypes, each of which is associated with a probability of occurrence or a frequency of occurrence and/or with a genetic value.

If desired, one or more of steps ST502 through ST507 in Figure 5 can be repeated one or more times in step ST508 in Figure 5 to generate one or more additional subsequent generations and/or further generations.

As shown in step ST510 in Figure 5, the distributions calculated in one or more iterations of step ST506 are compared to each other.

As shown in step ST512 in Figure 5, a breeding pair is chosen based on comparing step ST510.

As shown in step ST514 in Figure 5, the breeding pair and subsequent generations (if employed) are bred in accordance with the calculated or simulated breedings as set forth in steps ST506 and ST508.

As shown in step ST516 in Figure 5, a progeny individual having a desired genotype is identified.

## VII. Additional Considerations

For each of the methods disclosed herein, the methods can also further comprise generating one or more further generation progeny, wherein each further generation progeny is generated by one or more rounds of calculating, simulating, or combinations of calculating and simulating a breeding of at least one member of the subsequent generation or a later generation with an individual selected from the group consisting of itself, a member of the immediately prior generation, another individual from the same generation, another individual from a previous generation, the first breeding partner, the second breeding partner, and doubled haploid derivatives thereof. Strategies that can be employed for generating the further generation(s) can include, but are not limited to one or more successive generations of crossings, selfings, doubled haploid derivative generation, or combinations thereof of one or more individuals from a preceding generation, (e.g., one, two, three, four, or more successive generations of such crossings, selfings, doubled haploid derivative generation, or combinations thereof); at least one, two, three, four, or more successive generations of selfing of one or more members of a preceding generation.

The presently disclosed subject matter also encompasses individuals generated by the presently disclosed methods, as well as cells, parts, tissues, gametes, and progeny thereof. In some embodiments, the individuals are plants.

EXAMPLES

The presently disclosed subject matter will be now be described more fully hereinafter with reference to the accompanying Examples, in which exemplary embodiments of the presently disclosed subject matter are shown. The presently disclosed subject matter can, however, be embodied in different forms and should not be construed as limited to the embodiments set forth herein. Rather, these embodiments are provided so that this disclosure will be thorough and complete, and will fully convey the scope of the presently disclosed subject matter to those skilled in the art.

*Introduction to the EXAMPLES*

The methods disclosed herein are exemplified by an application of the presently disclosed subject matter in a maize breeding program described in EXAMPLES 1-9 and in a wheat breeding program described in EXAMPLES 10-17.

EXAMPLE 1 Plant Material - Maize

Parental material included two maize inbred lines: BFP57 and BMP34, both from the Stiff-Stalk Synthetic heterotic group. These lines were crossed with one another to produce $F_1$ seed. $F_1$ kernels were planted and the resulting $F_1$ plants were self-fertilized to produce $F_2$ seed. About 500 $F_2$ kernels were planted. The resulting $F_2$ plants were self-fertilized to produce $F_3$ seed.

One and only one $F_3$ kernel was harvested on each $F_2$ plant, a commonly-used generation advancement procedure known as single kernel descent (SKD). The almost 500 $F_3$ kernels so harvested were planted, and the resulting $F_3$ plants self-fertilized to produce $F_4$ seed. All $F_4$ kernels produced on each $F_3$ plant were harvested, keeping all $F_4$ kernels harvested separated by $F_3$ plant of origin, and thereby constituting $F_4$ families.

About 10 kernels from each $F_4$ family were planted to collect leaf tissue later used for DNA extraction and genotyping.

About 25 kernels from 250 unselected $F_4$ families were planted in an isolated field to be crossed to a tester (a maize inbred line from a different heterotic group than that of the two parental inbred lines of the project): BMT505, from the Lancaster heterotic group. $F_4$ plants were de-tasseled and thereby used as females, while the tester was used as the male to pollinate all $F_4$ plants. Testcross seed was harvested, maintaining the family structure.

EXAMPLE 2 Phenotypic Evaluations - Maize

Testcross seed from 229 $F_4$ families was planted at 6 field locations in two-row plots. The experimental design was a lattice design with one replication. Several other hybrids, used as checks, were also planted in the same trials.

Seed from the same 229 $F_4$ families was also planted at one additional field location, in one-row plots. Several inbred lines, used as checks, were also planted at the same location.

Traits measured included grain yield, grain moisture at harvest, root lodging, common smut incidence, and *Helminthosporium* incidence. Traits such as grain yield and grain moisture at harvest were only measured on testcross plots while others were measured either on testcross or $F_4$ plots, depending on their occurrence.

EXAMPLE 3 Genotyping and QTL Mapping - Maize

DNA was extracted from bulks of leaves of about 10 $F_4$ plants for each $F_4$ family. DNA samples were genotyped using 88 polymorphic SSR's covering the entire maize genome. Several hundred SSR's had been previously run on the two parents of this segregating population, BFP57 and BMP34, in order to identify the polymorphic ones. The molecular marker genotypes obtained from analyses of $F_4$ DNA bulks represented the genotypes of the $F_3$ plants from which $F_4$ families had been derived.

A molecular marker map was constructed using the commonly used software MapMaker and JoinMap. This molecular marker map had a total length of 1674 centiMorgans (cM), with a marker density of one marker every 19 cM.

Joint-analysis of genotypic and phenotypic data was performed using the software QTLCartographer and PlabQTL. Sixty-one QTLs were identified, for all traits. In particular, 14 QTLs were identified for grain yield, and 17 for grain moisture. QTLs are characterized by their position on the genetic map, and their additive and dominance effects. Positions are defined as a genetic distances between the most likely position of the QTLs (usually the position of the peak LOD score value) and flanking marker loci (in cM). Additive and dominance effects are defined as deviations from the mean and are expressed in the same unit as the trait they refer to. Additive values define which of the two parental lines carries the favorable allele at the QTL. In this case, additive values represent the effect of the BMP34 allele, whether positive or negative. For a trait such as grain yield where the desired effect is a higher value of the trait, a positive additive value means that BMP34 carries the favorable allele while a negative additive value means that BFP57 carries the favorable allele.

EXAMPLE 4 Selection Indices, Genetic Values, and Ideal Genotype - Maize

Based on the QTLs identified, selection indices were defined. These selection indices were then applied to the plants' QTL genotypes, to compute these plants' genetic values. Genetic value (GV) of a plant was computed as follows, based on the plant's QTL genotype:

$$GV = \sum_t \beta_t \sum_q \alpha_{qt} \sum_i p_{iqt} \delta_{iqt}$$

where $\beta_t$ is the weight (economic value) of trait t, $\alpha_{qt}$ is the effect of the favorable allele at QTL q of trait t (usually the additive value of the QTL), $p_{iqt}$ is the probability of occurrence of genotype i at QTL q of trait t, and $\delta_{iqt}$ is the selection value of QTL genotype i at QTL q of trait t. $\sum_i p_{iqt} \delta_{iqt}$ can be considered as the QTL genetic value (QTL q of trait t).

$\sum_q \alpha_{qt} \sum_i p_{iqt} \delta_{iqt}$ can be considered as the trait value (trait t).

In a segregating population of type $F_n$, which is the case of this population (n = 3), there are three possible genotypes at every QTL, namely QQ, Qq, and qq, where Q denotes the favorable and q the unfavorable alleles. Since QTLs are generally not located exactly at marker loci, exact genotypes at QTLs are not known. Nevertheless, QTL genotypes and their probabilities of occurrence, $p_{iqt}$, can be inferred from the genotypes of marker loci flanking the QTLs and plant ancestries (pedigrees) where i takes the values 1, 2, and 3, representing QTL genotypes QQ, Qq, and qq, as follows:

$$i = 1 \quad (QQ)$$

$$i = 2 \quad (Qq)$$

$$i = 3 \quad (qq)$$

Selection values of the QTL genotypes can be given arbitrary values. Most commonly, they take the following values:

$$\delta_{1qt} = 1$$

$$\delta_{2qt} = 0$$

$$\delta_{3qt} = -1$$

Several selection indices were built, involving more or fewer traits. An ideal genotype can be defined for each selection index. It is the genotype having homozygous favorable alleles at all QTL involved in the index.

One index (called IND) was based on 14 QTL for grain yield, 17 QTL for grain moisture at harvest, 13 QTL for root lodging, 7 QTL for common smut incidence, and 5 QTL for *Helminthosporium* incidence. QTL parameters were as defined in Tables 1-5 below. Additive effects were used as allele effects ($\alpha_{qt}$). Trait weights ($\beta_t$) were 1.2 for grain yield, -8.5 for grain moisture at harvest, -1.2 for root lodging, -9.6 for common smut incidence, and -78.1 for *Helminthosporium* incidence.

Table 1 QTL for Grain Yield

| QTL position | | QTL effect |
|---|---|---|
| Marker locus to the left of the QTL | Distance from marker locus to QTL (cM) | Additive effect |
| NOM0906 | 8.1 | -6.92 |
| NOM0538 | 2.0 | 3.59 |
| NOM0102 | 0.1 | -3.45 |
| NOM0544 | 10.5 | -11.13 |
| NOM0589 | 0.1 | -5.03 |
| NOM0099 | 24.0 | 24.95 |
| NOM0472 | 5.1 | 2.97 |
| NOM0181 | 0.3 | -2.05 |
| NOM0290 | 8.0 | -2.99 |
| NOM1024 | 9.0 | -4.08 |
| NOM0435 | 7.9 | 3.60 |
| NOM0130 | 0.5 | -5.09 |
| NOM0404 | 0.1 | 2.17 |
| NOM0548 | 0.3 | 2.52 |

Table 2 QTL for Grain Moisture at Harvest

| QTL position | | QTL effect |
|---|---|---|
| Marker locus to the left of the QTL | Distance from marker locus to QTL (cM) | Additive effect |
| NOM0533 | 1.5 | 0.57 |
| NOM0612 | 12.4 | -0.39 |
| NOM0129 | 6.0 | -0.28 |
| NOM0875 | 0.3 | -0.38 |
| NOM0359 | 10.7 | 0.94 |
| NOM0180 | 1.7 | -0.32 |
| NOM0499 | 13.2 | -2.63 |
| NOM0041 | 6.7 | 1.41 |
| NOM0528 | 0.1 | 0.35 |
| NOM0102 | 0.1 | -1.20 |
| NOM0296 | 5.1 | -0.42 |
| NOM0504 | 6.0 | 1.18 |
| NOM0732 | 8.1 | 0.25 |
| NOM0325 | 0.1 | 0.21 |

## EP 1 962 212 A1

(continued)

| QTL position | | QTL effect |
| --- | --- | --- |
| Marker locus to the left of the QTL | Distance from marker locus to QTL (cM) | Additive effect |
| NOM0561 | 0.2 | 0.19 |
| NOM0152 | 0.1 | 0.52 |
| NOM0612 | 11.6 | -0.49 |

Table 3 QTL for Root Lodging

| QTL position | | QTL effect |
| --- | --- | --- |
| Marker locus to the left of the QTL | Distance from marker locus to QTL (cM) | Additive effect |
| NOM0112 | 0.1 | 1.20 |
| NOM0218 | 2.3 | -3.76 |
| NOM0668 | 0.1 | 1.49 |
| NOM0290 | 22.0 | -11.60 |
| NOM0148 | 2.4 | -16.32 |
| NOM0021 | 27.4 | -28.40 |
| NOM0500 | 0.1 | -5.25 |
| NOM0329 | 7.8 | 1.13 |
| NOM0269 | 4.0 | 0.96 |
| NOM0538 | 2.0 | 4.67 |
| NOM0102 | 7.1 | -3.99 |
| NOM0021 | 2.6 | -6.50 |
| NOM0639 | 3.0 | 0.61 |

Table 4 QTLs for Common Smut Incidence

| QTL position | | QTL effect |
| --- | --- | --- |
| Marker locus to the left of the QTL | Distance from marker locus to QTL | Additive effect |
| NOM0435 | 2.0 | 1.97 |
| NOM1024 | -5.0 | 2.58 |
| NOM0097 | -4.0 | -1.13 |
| NOM0304 | -6.9 | -1.15 |
| NOM0296 | 5.9 | -1.04 |
| NOM0218 | 3.7 | 1.20 |
| NOM0324 | -6.3 | -1.34 |

Table 5 QTLs for *Helminthosporium* Incidence

| QTL position | | QTL effect |
| --- | --- | --- |
| Marker locus to the left of the QTL | Distance from marker locus to QTL | Additive effect |
| NOM0404 | 4.3 | -0.29 |

(continued)

| QTL position | | QTL effect |
|---|---|---|
| Marker locus to the left of the QTL | Distance from marker locus to QTL | Additive effect |
| NOM0528 | 4.0 | -0.29 |
| NOM0129 | 10.0 | 0.09 |
| NOM0329 | -2.2 | -0.30 |
| NOM0399 | 0.1 | -0.31 |

Genetic values for index IND were computed for all 229 $F_3$ plants for which genotypes had been previously obtained. None of the 229 $F_3$ plants matched the ideal genotype.

EXAMPLE 5 Predicted Distributions of Genetic Values - Maize

It was apparent, from the genotypes of these 229 $F_3$ plants that the ideal genotype could be obtained by successive cycles of crosses among plants. From these 229 $F_3$ plants, however, 26,106 non-reciprocal crosses can theoretically be made. Practically only 229 crosses can be made, given that each plant produces on average only one ear. Which are the best 229 crosses out of the 26,106 theoretically possible ones is the question that needed to be answered. Each cross, if made, would produce a number of different genotypes. These genotypes and their probability of occurrence can be computed from the genotypes of the plants to be crossed. Marker genotypes of the 229 $F_3$ plants were known therefore whole-genome marker genotypes of the potential progeny of crosses among these $F_3$ plants can be predicted. The probability of occurrence of each one of these whole-genome progeny genotypes can be computed from recombination distances between marker loci provided by the genetic map. Index values of these whole-genome progeny genotypes can also be computed. Once these index values are taken into consideration with their probabilities of occurrence, frequency distributions of index values of progenies can be constructed. These frequency distributions can be used to identify breedings (self-fertilizations or crosses) with high probabilities of generating high genetic value progeny. Quantile values of the frequency distributions are used to compared distributions and identify superior breedings.

EXAMPLE 6 Marker-Based Selection - Maize Round 1

The first round of marker-based selection operated on $F_3$ plants, for which marker genotypes were generated for the QTL mapping step. Since $F_3$ plants were not any longer available, hypothetical crosses (or selfs) among $F_4$ families were evaluated by computing frequency distributions of their progeny's genetic values and the associated quantile values. Seven different indices were used in the selection process.
Any hypothetical cross (self) that showed a negative 50% quantile value, for any index, was discarded resulting in 6,145 crosses being pre-selected. Pre-selected hypothetical crosses (selfs) with the highest values for the two most important indices were further selected, resulting in 126 final selections. An assessment of the $F_3$ plants involved in the selected crosses (selfs) allowed for the identification of the 12 $F_3$ plants involved in the largest number of highest value hypothetical crosses (selfs). This completed the first round of marker-based selection. Since $F_3$ plants were not available any longer $F_3$ progeny of these 12 selected $F_3$ plants was used to initiate the second round of marker-based selection.

EXAMPLE 7 Marker-Based Selection - Maize Round 2

About 45 kernels of each of the 12 selected $F_4$ families were planted, leaf sampled and genotyped with molecular markers flanking QTLs involved in the selection indices. There were a total of 531 $F_4$ plants.
Selection of $F_4$ plants proceeded in a similar manner as selection of $F_3$ plants. Hypothetical crosses (selfs) among the 531 $F_4$ plants were generated, the genetic value and frequency of occurrence of their progeny computed, and frequency distributions constructed and their quantile values computed. These calculations were done for each of the seven indices (the same as in Round 1) used in the selection process. Any hypothetical cross (self) that showed a negative 50% quantile value, for any index, was discarded. This resulted in about 60,000 hypothetical crosses being pre-selected. Genetically similar crosses (selfs), *i.e.* involving $F_4$ plants from the same two $F_4$ families (or single $F_4$ family in case of selfs) were identified and those with low quantile values were discarded. After this step only 4,073 hypothetical crosses (selfs) were still being considered for further evaluation. Hypothetical crosses (selfs) with the highest values for the two most important indices were further selected, resulting in 285 final selections. These 285 hypothetical crosses (selfs) involved 130 $F_4$ plants. Those plants were transplanted in the greenhouse and grown to maturity. Crosses (selfs) among plants were made based on their value and male-female flowering synchrony / asynchrony of the plants. A total of 130 crosses and selfs were made, representing the best set of crosses that could practically be realized. Seed ($C_1$ seed) from the nine best crosses ($C_1$ families) was harvested to initiate the next round of selection. Some seed of these nine best crosses as well as seed of the other 121 crosses (selfs) was delivered to maize breeders for further phenotypic evaluation, selection, and advancement.

EXAMPLE 8 Marker-Based Selection - Maize Round 3

A total of 551 kernels from the 9 selected $C_1$ families were planted, leaf sampled and genotyped with molecular markers flanking QTLs involved in the selection indices.

Selection of $C_1$ plants proceeded in a similar manner as selection of $F_4$ plants. Hypothetical crosses (selfs) among the 551 $C_1$ plants were generated, the genetic value and frequency of occurrence of their progeny computed, and frequency distributions constructed and their quantile values computed. These calculations were done for each of the seven indices (the same as in previous rounds) used in the selection process. Any hypothetical cross (self) that showed a negative 50% quantile value, for any index, was discarded. This resulted in about 60,000 hypothetical crosses being pre-selected. Genetically similar crosses (selfs), *i.e.* involving $C_1$ plants from the same two $C_1$ families (or single $C_1$ family in case of selfs) were identified and those with low quantile values were discarded. After this step only 2,438 hypothetical crosses (selfs) were still being considered for further evaluation. Hypothetical crosses (selfs) with the highest values for the two most important indices were further selected, resulting in 309 final selections. These 309 hypothetical crosses (selfs) involved 141 $C_1$ plants. Those plants were transplanted in the greenhouse and grown to maturity. Crosses (selfs) among plants were made based on their value and male-female flowering synchrony / asynchrony of the plants. A total of 141 crosses and selfs were made, representing the best set of crosses that could practically be realized. Seed ($C_2$ seed) from the nine best crosses ($C_2$ families) was harvested to initiate the next round of selection. Some seed of these nine best crosses as well as seed of the other 132 crosses (selfs) was delivered to maize breeders for further phenotypic evaluation, selection, and advancement.

EXAMPLE 9 Marker-Based Selection - Maize Round 4

A total of 519 kernels from the 9 selected $C_2$ families were planted, leaf sampled and genotyped with molecular markers flanking QTLs involved in the selection indices.

Selection of $C_2$ plants proceeded in a similar manner as selection of $C_1$ plants. Hypothetical crosses (selfs) among the 519 $C_2$ plants were generated, the genetic value and frequency of occurrence of their progeny computed, and frequency distributions constructed and their quantile values computed. These calculations were done for each of the seven indices (the same as in previous rounds) used in the selection process. Any hypothetical cross (self) that showed a negative 50% quantile value, for any index, was discarded. This resulted in about 55,000 hypothetical crosses being pre-selected. Genetically similar crosses (selfs), *i.e.* involving $C_2$ plants from the same two $C_2$ families (or single $C_2$ family in case of selfs) were identified and those with low quantile values were discarded. After this step only 1,696 hypothetical crosses (selfs) were still being considered for further evaluation. Hypothetical crosses (selfs) with the highest values for the two most important indices were further selected, resulting in 163 final selections. These 163 hypothetical crosses (selfs) involved 120 $C_2$ plants. Those plants were transplanted in the greenhouse and grown to maturity. Crosses (selfs) among plants were made based on their value and male-female flowering synchrony / asynchrony of the plants. A total of 120 crosses and selfs were made, representing the best set of crosses that could practically be realized. Seed ($C_3$ seed) from these 120 crosses and selfs ($C_3$ families) was harvested and delivered to maize breeders for further phenotypic evaluation, selection, and advancement.

Representative results of the Marker-Based Selections disclosed in EXAMPLES 6-9 are provided in Figures 6 and 7, wherein individuals MDL53 and MDL54 were produced by employing the methods disclosed herein.

EXAMPLE 10 Plant Material - Wheat

A segregating population was created from crossing two wheat inbred lines, BR25 and FO71. Several plants of one line were crossed with several plants of the other line to produce $F_1$ seed. $F_1$ kernels were planted. The resulting $F_1$ plants were self-fertilized to produce $F_2$ seed. About 400 $F_2$ kernels were planted and $F_2$ plants were self-fertilized to produce $F_3$ seed.

One and only one $F_3$ kernel was harvested on each $F_2$ plant, a commonly-used generation advancement procedure known as single kernel descent (SKD) resulting in a bulk of 400 $F_3$ kernels. These 400 $F_3$ kernels were planted, and $F_3$ plants self-fertilized to produce $F_4$ seed. All $F_4$ kernels produced on each $F_3$ plant were harvested, keeping all $F_4$ kernels harvested separated by $F_3$ plant of origin, and thereby constituting $F_4$ families (400).

One row kernels of each $F_4$ family was planted and $F_4$ plants self-fertilized in order to increase seed quantities.

The harvested seed consisted of the $F_5$ generation.

All $F_5$ kernels were harvested in bulk on each $F_4$ row (one bulk per row). In the end of this process 400 so-called $F_3$:$F_5$ families were available. Leaf tissue of each $F_3$:$F_5$ family was sampled by bulking leaf disk samples from 12 $F_5$ plants per $F_3$:$F_5$ family. These leaf samples were later used for DNA extraction and genotyping. The genotyped obtained represented the genotypes of the $F_3$ plants.

EXAMPLE 11 Phenotypic Evaluations - Wheat

The 400 $F_5$ families were evaluated phenotypically in field trials conducted in 2002 (1 location in France) and in 2003 (4 locations in France, 1 in Germany and 1 in the United Kingdom). The experimental design was a randomized complete block design with repeated checks. Parental lines as well as several other lines were used as checks and were therefore planted in the same trials.

The following traits were evaluated: grain yield, heading date, lodging, yellow rust incidence, eyespot incidence, thousand-kernel weight (TKW), test-weight, hardness, protein content, SDS sedimentation test, Mixograph parameters, and high molecular weight glutenin subunits.

EXAMPLE 12 Genotyping and QTL Mapping - Wheat

DNA was extracted from bulks of leaves of about 12 $F_5$ plants for each $F_4$ family. DNA samples were genotyped using 170 SSRs covering the entire wheat genome. The two parental lines of this segregating population, BR25 and FO71, had previously been genotyped at several hundred SSR markers in order to identify polymorphisms between them. The molecular marker genotypes obtained from analyses of $F_5$ DNA bulks represented the genotypes of the $F_3$ plants from which $F_4$ and $F_5$ families had been derived.

A molecular marker map was constructed using the commonly used software Mapmaker. Joint-analysis of genotypic and phenotypic data was performed using the software QTLCartographer and PlabQTL. More than fifty QTLs were identified for all traits. In particular, 11 QTLs were identified for grain yield, and 12 for the SDS sedimentation test. QTLs were characterized by their position on the genetic map, and their additive effect. Positions were defined as genetic distances (in centimorgans - cM) between the most likely position of the QTLs (usually the position of the peak LOD score value) and flanking marker loci. Additive effects are defined as deviations from the mean and are expressed in the same unit as the trait they refer to. Additive values define which of the two parental lines carries the favorable allele at the QTL. In this case, additive values represent the effect of the allele carried by FO71, whether positive or negative. For a trait such as grain yield where the desired effect is a higher value of the trait, a positive additive value means that FO71 carries the favorable allele and BR25 the unfavourable one. Similarly, a negative additive value means that BR25 carries the favorable allele and FO71 the unfavourable one.

EXAMPLE 13 Selection Indices, Genetic Values, and Ideal Genotype - Wheat

Based on the QTLs identified, selection indices were defined. These selection indices were then applied to the plants' QTL genotypes, to compute these plants' genetic values. Genetic value (GV) of a plant was computed as follows, based on the plant's QTL genotype:

$$GV = \sum_t \beta_t \sum_q \alpha_{qt} \sum_i p_{iqt} \delta_{iqt}$$

where $\beta_t$ is the weight (economic value) of trait t, $\alpha_{qt}$ is the effect of the favorable allele at QTL q of trait t (usually the additive value of the QTL), $p_{iqt}$ is the probability of occurrence of genotype i at QTL q of trait t, and $\delta_{iqt}$ is the selection value of QTL genotype i at QTL q of trait t.

$\displaystyle\sum_i p_{iqt} \delta_{iqt}$ can be considered as the QTL genetic value (QTL q of trait t).

$\displaystyle\sum_q \alpha_{qt} \sum_i p_{iqt} \delta_{iqt}$ can be considered as the trait value (trait t).

In a segregating population of type $F_n$, which is the case of this population (n = 3), there are three possible genotypes at every QTL, namely QQ, Qq, and qq, where Q denotes the favorable and q the unfavorable alleles. Since QTLs are generally not located exactly at marker loci, exact genotypes at QTLs are not known. Nevertheless, QTL genotypes and their probabilities of occurrence, $p_{iqt}$, can be inferred from the genotypes of marker loci flanking the QTLs and plant ancestries (pedigrees) where i takes the values 1, 2, and 3, representing QTL genotypes QQ, Qq, and qq, as follows:

$$i = 1 \quad (QQ)$$

$$i = 2 \quad (Qq)$$

$$i = 3 \quad (qq)$$

Selection values of the QTL genotypes can be given arbitrary values. In this example selection values of the QTLs were assigned the following values:

$$\delta_{1qt} = 1$$

$$\delta_{2qt} = 0$$

$$\delta_{3qt} = -1$$

Several selection indices were built, involving more or fewer traits. An ideal genotype can be defined for each selection index. It is the genotype having homozygous favorable alleles at all QTL involved in the index.

One index (called IND) was based on 11 QTLs for grain yield, 12 QTLs for the SDS sedimentation test, 12 for protein content and 15 for TKW. QTL parameters were as defined below. Allele effects ($\alpha_{qt}$) were set to equal the additive effect values. Trait weights ($\beta_t$) were 2.7 for grain yield, -10 for the SDS sedimentation test, -3 for protein content, and -15 for TKW.

Table 6 QTLs for Grain Yield

| QTL position | | | QTL effect |
|---|---|---|---|
| Linked marker locus | Chromosome | Genetic map position (cM) | Additive effect |
| NW1105 | 1 B-2 | 20 | -1.62 |
| NW0757 | 2A-1 | 0 | 2.17 |
| NW0641 | 2A-2 | 98 | 1.42 |
| NW1425 | 2A-3 | 0 | 1.65 |
| NW1574 | 3A | 114 | -2.08 |
| NW1736 | 3B-1 | 18 | 1.67 |
| NW1430 | 3D | 58 | 0.95 |
| NW1585 | 5B | 72 | -1.46 |
| NW0071 | 6A | 36 | 1.44 |
| DW0370 | 6B | 0 | -0.37 |
| NW0508 | 7A | 136 | 1.00 |

Table 7 QTLs for Thousand-Kernel Weight (TKW)

| QTL position | | | QTL effect |
|---|---|---|---|
| Linked marker locus | Chromosome | Genetic map position (cM) | Additive effect |
| NW0440 | 1 A-1 | 50 | -0.58 |
| NW0758 | 2A-2 | 10 | -0.65 |
| NW117A | 2D-3 | 16 | -0.45 |
| NW1583 | 3A | 26 | 0.66 |
| NW1821 | 3B-1 | 6 | 0.45 |
| DW0955 | 3B-2 | 8 | -0.45 |
| NW2009 | 5A | 10 | 0.57 |
| NW1648 | 5B | 0 | 0.57 |
| NW1585 | 5B | 70 | -0.73 |
| NW1651 | 5D-1 | 104 | 0.67 |
| NW0071 | 6A | 32 | 1.34 |
| NW2870 | 6B | 4 | 0.74 |
| NW1197 | 6D | 26 | 0.50 |
| NW1034 | 7A | 30 | -0.56 |
| NW1295 | 7D-3 | 0 | -0.96 |

Table 8 QTLs for Protein Content

| QTL position | | | QTL effect |
|---|---|---|---|
| Linked marker locus | Chromosome | Genetic map position (cM) | Additive effect |
| NW1074 | 1 A-1 | 18 | 0.09 |
| NW1105 | 1 B-2 | 22 | -0.08 |
| NW0814 | 2A-2 | 108 | -0.21 |
| NW1425 | 2A-3 | 0 | -0.10 |
| NW01 80 | 2D-2 | 2 | -0.12 |
| NW0790 | 3A | 102 | 0.21 |
| DW 1718 | 3B-2 | 0 | -0.09 |
| NW0659 | 5A | 6 | -0.08 |
| NW0692 | 5D-1 | 0 | -0.12 |
| NW0071 | 6A | 29 | -0.13 |
| NW1673 | 7D-2 | 68 | 0.07 |
| NW1475 | 7D-4A | 8 | -0.14 |

Table 9 QTLs for the SDS Sedimentation Test

| QTL position | | | QTL effect |
|---|---|---|---|
| Linked marker locus | Chromosome | Genetic map position (cM) | Additive effect |
| NW0151 | 1A-1 | 0 | -0.81 |
| NW1272 | 1 A-1 | 62 | -1.59 |
| NW1105 | 1 B-2 | 22 | -2.64 |
| NW0222 | 3A | 126 | -1.39 |
| NW1736 | 3B-1 | 14 | 1.23 |
| DW 1718 | 3B-2 | 0 | -0.85 |
| NW0692 | 5D-1 | 0 | -3.04 |
| DW0935 | 5D-2 | 42 | 1.41 |
| NW0718 | 6B | 32 | 1.98 |
| NW1034 | 7A | 26 | -0.66 |
| NW0779 | 7A | 81 | 1.39 |
| NW1475 | 7D-4A | 0 | -1.75 |

Genetic values for index IND were computed for all 400 $F_3$ plants for which genotypes had been previously obtained. None of the plants matched the ideal genotype.

EXAMPLE 14 Predicted Distributions of Genetic Values - Wheat

The genotypes of these 400 $F_3$ plants indicated that the ideal genotype could be obtained by successive cycles of crosses among plants. The challenge was to identify the crosses, from all possible ones, which would be the best crosses in terms of allowing individuals having genotypes identical or similar to that of the ideal genotype to develop. Each cross, if made, would produce a number of different genotypes. These genotypes and their probability of occurrence can be computed from the genotypes of the plants to be crossed. Marker genotypes of the 400 $F_3$ plants were known therefore whole-genome marker genotypes of the potential progeny of crosses among these $F_3$ plants can be predicted. The probability of occurrence of each one of these whole-genome progeny genotypes can be computed from recombination distances between marker loci provided by the genetic map. Index values of these whole-genome progeny genotypes can also be computed. Once these index vales are taken into consideration with their probabilities of occurrence, frequency distributions of index values of progenies can be constructed. These frequency distributions can be used to identify matings (self-fertilizations or crosses) with high probabilities of generating high genetic value progeny. Quantile values of the frequency distributions are used to compared distributions and identify superior matings.

EXAMPLE 15 Marker-Based Selection - Wheat Round 1

The first round of marker-based selection operated on $F_3$ plants, for which marker genotypes were generated for the QTL mapping step. Since $F_3$ plants were not any longer available, hypothetical crosses (or selfs) among $F_4$ or $F_5$ families were evaluated by computing frequency distributions of their progeny's genetic values and the associated quantile values. One index, IND was used in the selection process.
Any hypothetical cross (self) that showed a negative 50% quantile value for index IND was discarded resulting in several hypothetical crosses being pre-selected. Pre-selected hypothetical crosses (selfs) with the highest values for index IND were further selected, resulting in 40 final selections. An assessment of the $F_3$ plants involved in the selected crosses (selfs) allowed for the identification of the 15 $F_3$ plants involved in the largest number of highest value hypothetical crosses (selfs). This completed the first round of marker-based selection. Since $F_3$ plants were not available any longer $F_5$ progeny of these 15 selected $F_3$ plants was used to initiate the second round of marker-based selection.

EXAMPLE 16 Marker-Based Selection - Wheat Round 2

About 28 kernels of each of the 15 selected $F_5$ families were planted, leaf sampled and genotyped with molecular markers flanking QTLs involved in the selection indices. There were a total of 420 $F_5$ plants.

Selection of $F_5$ plants proceeded in a similar manner to the selection of $F_3$ plants. Hypothetical crosses (selfs) among the 420 $F_5$ plants were generated, the genetic value and frequency of occurrence of their progeny computed, and frequency distributions constructed and their quantile values computed. These calculations were done for index IND, used in the selection process. Any hypothetical cross (self) that showed a negative 50% quantile valuefor index IND was discarded. Genetically similar crosses (selfs), *i.e.* involving $F_5$ plants from the same two $F_5$ families (or single $F_5$ family in case of selfs) were identified and those with low quantile values were discarded. After this step, only around 4,000 hypothetical crosses (selfs) were still being considered for further evaluation. Hypothetical crosses (selfs) with the highest values for the two most important indices were further selected, resulting in 40 final selections. These 40 hypothetical crosses (selfs) involved 50 $F_5$ plants. Those plants were transplanted in the greenhouse and grown to maturity. Crosses (selfs) among plants were made based on their value and male-female flowering synchrony / asynchrony of the plants. A total of 35 crosses and selfs were made, representing the best set of crosses that could practically be realized. Seed ($C_1$ seed) from the 18 best crosses ($C_1$ families) was harvested to initiate the next round of selection. Some seed of these best crosses as well as seed of the other crosses (selfs) was delivered to wheat breeders for further phenotypic evaluation, selection, and advancement.

EXAMPLE 17 Marker-Based Selection - Wheat Round 3

A total of 540 kernels from the 18 selected $C_1$ families were planted, leaf sampled and genotyped with molecular markers flanking QTLs involved in the selection index.

Selection of $C_1$ plants proceeded in a similar manner as selection of $F_5$ plants. Hypothetical crosses (selfs) among the 540 $C_1$ plants were generated, the genetic value and frequency of occurrence of their progeny computed, and frequency distributions constructed and their quantile values computed. These calculations were done for index IND, used in the selection process. Any hypothetical cross (self) that showed a negative 50% quantile value for index IND was discarded Genetically similar crosses (selfs), *i.e.* involving $C_1$ plants from the same two $C_1$ families (or single $C_1$ family in case of selfs) were identified and those with low quantile values were discarded. After this step, only around 3,000 hypothetical crosses (selfs) were still being considered for further evaluation. Hypothetical crosses (selfs) with the highest values for index IND were further selected, resulting in 40 final selections. These 40 hypothetical crosses (selfs) involved 45 $C_1$ plants. Those plants were transplanted in the greenhouse and grown to maturity. A total of 36 crosses and selfs were made, representing the best set of crosses that could practically be realized. Seed ($C_2$ seed) from the all crosses and selfs ($C_2$ families) were harvested and delivered to wheat breeders for further seed increase, phenotypic evaluation, selection, and advancement.

*Discussion of the EXAMPLES*

The presently disclosed subject matter relates in some embodiments to the selection of plants to be crossed or selfed based on the characteristics of their potential progeny. Progeny characteristics include their individual genotypes, probabilities of occurrence of these individual genotypes, and genetic values of these genotypes, as well as overall progeny characteristics such as the frequency distribution of genetic values and corresponding quantile values. Progeny characteristics can be calculated rather than estimated through simulation. Progeny can be the immediate product of a specific cross or self or the product of a specific cross or self followed by several generations of self-fertilizing or crossing.

Marker-trait associations are not limited to QTL but also include genes. For marker-trait associations or gene information to be useable through the presently disclosed subject matter, availability of genetic map information and sequence polymorphism is desirable.

The population to which the presently disclosed subject matter can be applied can be any type of population, in some embodiments a bi-parental (bi-allelic) population, although this is not necessary. Currently, various algorithms and software have been developed for the bi-allelic situation, but development of algorithms and software for multi-allelic situations are also provided in accordance with the presently disclosed subject matter. The population can be $F_2$ individuals or any $F_n$ generation. It can also be any $BC_n$ generation, recombinant inbred lines (RILs), near-isogenic lines (NILs), doubled-haploids (DHs), or any other material. C1 and C2 plants, as illustrated in the above EXAMPLES, constitute segregating populations where individuals can have either homozygous or heterozygous genotypes at any locus.

In the above EXAMPLES, the population of plants to which marker-based selection is applied can be the same generation as that used to establish marker-trait (genotype-phenotype) associations. The presently disclosed methods also apply to situations where the marker-trait associations have been established on populations independent from those where marker-based selection is applied. Marker-trait associations can even come from several independent populations. For instance, one might have conducted QTL mapping projects which have resulted in marker-trait associations. Published experiments run at public institutions might have also resulted in marker-trait associations. Finally, information about genes, including map positions and sequence polymorphism (haplotypes) might also be available. All this information, marker-trait associations from internal experiments, external experiments, as well as gene information, can be used to conduct marker-based selection in another population.

The number of consecutive generations to which the presently disclosed subject matter can be applied is unlimited.

Although the above EXAMPLES illustrate the application of a representative method for crossing or selfing plants within the population under study, the presently disclosed subject matter can also be employed for selecting plants to be backcrossed to a unique and homozygous line.

The number of individuals to which the presently disclosed subject matter is applied is unlimited.

The presently disclosed subject matter can be applied to any species, not limited to plants.

REFERENCES

All references listed in the instant disclosure, including but not limited to all patents, patent applications and publications thereof, scientific journal articles, and database entries (e.g., GENBANK® database entries and all annotations available therein) are incorporated herein by reference in their entireties to the extent that they supplement, explain, provide a background for, or teach methodology, techniques, and/or compositions employed herein.

Beavis (1994) in Wilkinson (ed.) Proc. 49th Ann Corn and Sorghum Res Conf, American Seed Trade Association, Chicago, Illinois, United States of America, pp 250-266.

Edwards et al. (1987) 115 Genetics 113-125.

Fisch et al. (1996) Genetics 143:571 577.

Jaccoud et al. (2001) 29 Nucleic Acids Res e25.

Lander & Schork (1994) 265 Science 2037-2048.

Stam (1994) in van Ooijen & Jansen (eds.) Biometrics in plant breeding: applications of molecular markers. Proc. 9th Meeting Eucarpia Section Biometrics. Plant Research International, Wageningen, the Netherlands.

U.S. Patent Application Publication No. 20030005479.

U.S. Patent Nos. 5,385,835; 5,492,547; 5,981,832; 6,399,855; 7,135,615.

Wan et al. (1989) Theoretical and Applied Genetics 77:889-892.

It will be understood that various details of the presently disclosed subject matter can be changed without departing from the scope of the presently disclosed subject matter. Furthermore, the foregoing description is for the purpose of illustration only, and not for the purpose of limitation.

**Claims**

1. A method for calculating a distribution of a probability or frequency of occurrence of one or more potential genotypes, the method comprising:

   (a) providing a first breeding partner and a second breeding partner, wherein:

   (i) the genotype of each of the first breeding partner and the second breeding partner is known or is predictable with respect to one or more genetic markers, each of which is linked to a genetic locus; and
   (ii) a genetic distance between each genetic marker and the genetic locus to which it is linked is known or can be assigned;

   (b) calculating, simulating, or combinations of calculating and simulating a breeding of the first breeding partner and the second breeding partner to generate a subsequent generation, each member of the subsequent generation comprising a genotype; and
   (c) calculating a distribution of a probability or a frequency of occurrence for one or more of the genotypes of one or more members of the subsequent generation.

2. A method for calculating a genetic value distribution, the method comprising:

   (a) providing a first breeding partner and a second breeding partner, wherein:

   (i) the genotype of each of the first breeding partner and the second breeding partner is known or is predictable with respect to one or more genetic markers linked to one or more genetic loci;
   (ii) a genetic distance between each genetic marker and the genetic locus to which it is linked is known or can be assigned; and
   (iii) each genotype is associated with a genetic value;

(b) calculating, simulating, or combinations of calculating and simulating a breeding of the first breeding partner and the second breeding partner to generate a subsequent generation, each member of the subsequent generation comprising a genotype; and
(c) calculating a genetic value distribution for one or more of the genotypes.

3. A method for choosing a breeding pair for producing a progeny having a desired genotype, the method comprising:

(a) providing a first breeding partner and a second breeding partner, wherein:

(i) the genotype of each of the first breeding partner and the second breeding partner is known or is predictable with respect to one or more genetic markers, each of which is linked to a genetic locus; and
(ii) a genetic distance between each genetic marker and the genetic locus to which it is linked is known or can be assigned;

(b) calculating, simulating, or combinations of calculating and simulating a breeding of the first breeding partner and the second breeding partner to generate a subsequent generation, each member of the subsequent generation comprising a genotype;
(c) calculating a distribution of a probability or a frequency of occurrence for one or more of the genotypes of one or more members of the subsequent generation;
(d) repeating steps (a) through (c) with a different first, different second, or both different first and different second potential breeding partners;
(e) comparing the probability or frequency distributions calculated in one or more iterations of step (c) to each other; and
(f) choosing a breeding pair based on the comparing step.

4. A method for choosing a breeding pair for producing a progeny having a desired genotype, the method comprising:

(a) providing a first breeding partner and a second breeding partner, wherein:

(i) the genotype of each of the first breeding partner and the second breeding partner is known or is predictable with respect to one or more genetic markers linked to one or more genetic loci;
(ii) a genetic distance between each genetic marker and the genetic locus to which it is linked is known or can be assigned; and
(iii) each genotype is associated with a genetic value;

(b) calculating, simulating, or combinations of calculating and simulating a breeding of the first breeding partner and the second breeding partner to generate a subsequent generation, each member of the subsequent generation comprising a genotype;
(c) calculating a distribution of genetic values associated with one or more of the genotypes of one or more members of the subsequent generation;
(d) repeating steps (a) through (c) with a different first, different second, or both different first and different second potential breeding partners;
(e) comparing the genetic value distributions calculated in one or more iterations of step (c) to each other; and
(f) choosing a breeding pair based on the comparing step.

5. A method for generating a progeny individual having a desired genotype, the method comprising:

(a) providing a first breeding partner and a second breeding partner, wherein:

(i) the genotype of each of the first breeding partner and the second breeding partner is known or is predictable with respect to one or more genetic markers, each of which is linked to a genetic locus; and
(ii) a genetic distance between each genetic marker and the genetic locus to which it is linked is known or can be assigned;

(b) calculating, simulating, or combinations of calculating and simulating a breeding of the first breeding partner and the second breeding partner to generate a subsequent generation, each member of the subsequent generation comprising a genotype;
(c) calculating a distribution of a probability or a frequency of occurrence for one or more of the genotypes of one or more members of the subsequent generation;

(d) repeating steps (a) through (c) with a different first, different second, or both different first and different second potential breeding partners;

(e) comparing the probability or frequency distributions calculated in one or more iterations of step (c) to each other;

(f) choosing a breeding pair based on the comparing step; and

(g) breeding the breeding pair in accordance with the calculating, simulating, or combinations of calculating and simulating as set forth in step (b) to generate a progeny individual having a desired genotype.

6. A method for generating a progeny individual having a desired genotype, the method comprising:

(a) providing a first breeding partner and a second breeding partner, wherein:

(i) the genotype of each of the first breeding partner and the second breeding partner is known or is predictable with respect to one or more genetic markers linked to one or more genetic loci;

(ii) a genetic distance between each genetic marker and the genetic locus to which it is linked is known or can be assigned; and

(iii) each genotype is associated with a genetic value;

(b) calculating, simulating, or combinations of calculating and simulating a breeding of the first breeding partner and the second breeding partner to generate a subsequent generation, each member of the subsequent generation comprising a genotype;

(c) calculating a distribution of genetic values associated with one or more of the genotypes of one or more members of the subsequent generation;

(d) repeating steps (a) through (c) with a different first, different second, or both different first and different second potential breeding partners;

(e) comparing the genetic value distributions calculated in one or more iterations of step (c) to each other;

(f) choosing a breeding pair based on the comparing step; and

(g) breeding the breeding pair in accordance with the calculating, simulating, or combinations of calculating and simulating as set forth in step (b) to generate a progeny individual having a desired genotype.

7. An individual generated by the method of one of claims 5 and 6.

8. The individual of claim 7, wherein the individual is a plant.

9. A cell from the plant of claim 8.

10. Seed or progeny from the plant of claim 8.

11. The method of one of claims 3-6, wherein the comparing is at a selected quantile.

12. The method of claim 11, wherein the selected quantile is a 95% quantile, a 50% quantile, or combinations thereof.

13. The method of one of claims 1-6, wherein each breeding partner is a plant.

14. The method of claim 13, wherein the plant is selected from the group consisting of maize, wheat, barley, rice, sugar beet, sunflower, winter oilseed rape, canola, tomato, pepper, melon, watermelon, broccoli, cauliflower, Brussel sprouts, lettuce, spinach, sugar cane, coffee, cocoa, pine, poplar, eucalyptus, apple tree, and grape.

15. The method of claim 14, wherein the plant is maize.

16. The method of one of claims 1-6, wherein each breeding partner is an inbred individual.

17. The method of one of claims 1-6, further comprising generating one or more further generation progeny, wherein each further generation progeny is generated by one or more rounds of calculating, simulating, or combinations of calculating and simulating a breeding of at least one member of the subsequent generation or a later generation with an individual selected from the group consisting of itself, a member of the immediately prior generation, another individual from the same generation, another individual from a previous generation, the first breeding partner, the second breeding partner, and doubled haploid derivatives thereof.

18. The method of claim 17, wherein the further generation progeny are generated by one or more successive generations of crossings, selfings, doubled haploid derivative generation, or combinations thereof of one or more individuals from a preceding generation.

19. The method of claim 18, wherein the further generation progeny are generated by three successive generations of crossings, selfings, doubled haploid derivative generation, or combinations thereof of one or more individuals of a preceding generation.

20. The method of claim 18, wherein the further generation progeny are generated by four successive generations of crossings, selfings, doubled haploid derivative generation, or combinations thereof of one or more individuals from a preceding generation.

21. The method of one of claims 1-6, wherein the further generation is generated by at least two successive generations of selfing of one or more members of a preceding generation.

22. The method of claim 21, wherein the further generation is generated by three successive generations of selfing of one or more members of a preceding generation.

23. The method of claim 22, wherein the further generation is generated by four successive generations of selfing of one or more members of a preceding generation.

24. The method of one of claims 1-6, wherein the one or more genetic markers are selected from the group consisting of a single nucleotide polymorphism (SNP), an indel (*i.e.*, insertion/deletion), a simple sequence repeat (SSR), a restriction fragment length polymorphism (RFLP), a random amplified polymorphic DNA (RAPD), a cleaved amplified polymorphic sequence (CAPS) marker, a Diversity Arrays Technology (DArT) marker, an amplified fragment length polymorphism (AFLP), and combinations thereof.

25. The method of one of claims 1-6, wherein the one or more genetic markers comprise between one and ten markers.

26. The method of one of claims 1-6, wherein the one or more genetic markers comprise more than ten genetic markers.

27. The method of one of claims 1-6, wherein the calculating, simulating, or combinations of calculating and simulating a breeding includes calculating, simulating, or combinations of calculating and simulating an expected rate of recombination between at least one of the one or more genetic markers and a genetic locus associated with expression of a phenotypic trait.

28. The method of claim 27, wherein the phenotypic trait is selected from the group consisting of a qualitative trait and a quantitative trait.

29. The method of claim 28, wherein the one or more genetic markers are linked to one or more quantitative trait loci associated with expression of the phenotypic trait.

30. The method of claim 27, wherein the genetic locus associated with expression of the phenotypic trait encodes a gene product that is associated with expression of the phenotypic trait.

31. The method of claim 27, wherein the rate of recombination between the at least one of the one or more genetic markers and the genetic locus associated with expression of the phenotypic trait is zero.

32. The method of one of claims 1-6, wherein the breeding partners are the same individual.

33. The method of one of claims 1-6, wherein each calculated or simulated breeding comprises selfing an individual from the immediately prior generation.

34. The method of one of claims 1-6, wherein the breeding pair comprises a pool of male genotypes, a pool of female genotypes, or both a pool of male and a pool of female genotypes.

**100**

*FIGURE 1*

EP 1 962 212 A1

**200**

Provide a first breeding partner and a second breeding partner
with known or predictable genotypes with respect to one or more genetic markers,
each of which is linked to a genetic locus — ST202

Establish a subsequent generation, each member of the
subsequent generation comprising a genotype — ST204

Add Further Generation(s)?

YES

NO

Generate one or more
further generations — ST205

ST206 — Calculate a distribution of a probability or a frequency of occurrence
for one or more of the genotypes of one or more members
of the subsequent and/or further generation(s)

Display Results — ST208

**FIGURE 2**

**300**

Provide a first breeding partner and a second breeding partner
with known or predictable genotypes with respect to one or more genetic markers,
each of which is linked to a genetic locus and
each genotype of which is associated with a genetic value — ST302

↓

Establish a subsequent generation, each member of the
subsequent generation comprising a genotype and
each genotype is associated with a genetic value — ST304

↓

Add Further
Generation(s)?

**YES** →

**NO** ↓

Generate one or more
further generations — ST305

ST306 — Calculate a genetic value distribution for
one or more of the genotypes

↓

Display Results — ST308

*FIGURE 3*

**400**

```
┌─────────────────────────────────────────────────────────────┐    ST402
│           Provide a first breeding partner and a second      │
│   breeding partner with known or predictable genotypes with  │
│   respect to one or more genetic markers, each of which is    │
│              linked to a genetic locus                        │
└─────────────────────────────────────────────────────────────┘
```

Add Genetic Values?

YES

NO

```
┌──────────────────────────────┐   ST403
│   Associate each genotype     │
│   with a genetic value        │
└──────────────────────────────┘
```

ST404 — Establish a subsequent generation, each member of the subsequent generation comprising a genotype

ST406 — Calculate a distribution of a probability or a frequency of occurrence and/or of a genetic value for one or more of the genotypes of one or more members of the subsequent generation

Add Further Generation(s)?

YES

NO

ST407 — Generate one or more further generations

ST408 — Repeat steps ST402 and ST404, optionally including step ST403 and/or step ST407, to establish one or more additional subsequent generations and/or further generations

ST410 — Compare the distributions calculated in one or more iterations of step ST406 to each other

ST412 — Choose a breeding pair based on comparing step ST410

*FIGURE 4*

**500**

Provide a first breeding partner and a second breeding partner with known or predictable genotypes with respect to one or more genetic markers, each of which is linked to a genetic locus ——ST502

Add Genetic Values? —— YES —→ Associate each genotype with a genetic value ——ST503

NO

ST504—— Establish a subsequent generation, each member of the subsequent generation comprising a genotype

ST506—— Calculate a distribution of a probability or a frequency of occurrence for one or more of the genotypes of one or more members of the subsequent generation

Add Further Generation(s)? —— YES —→ Generate one or more further generations ——ST507

NO

ST508—— Repeat steps ST502 through ST506, optionally including step ST503 and/or step ST507, to establish one or more additional subsequent generations and/or further generations

ST510—— Compare the distributions calculated in one or more iterations of step ST506 to each other

ST512—— Choose a breeding pair based on comparing step ST510

Breed the breeding pair chosen in step ST512 and subsequent and further generations in accordance with the calculated or simulated breedings as set forth in steps ST506 and ST508 ——ST514

Identify a progeny individual having a desired genotype ——ST516

*FIGURE 5*

FIGURE 6

Grain Moisture (%)

Grain Yield (Qx/ha)

122.3

114.0

105.7

97.4

22.0  23.2  24.4  25.6  26.8  28.0  29.2

□ CHECK

■ MDL53/T51

□ CHECK
□ CHECK  □ BFP57/T51  ■ MDL53/T42

■ MDL54/T51

■ MDL54/T58
■ MDL53/T58

■ MDL54/T42

□ BFP57/T42

■ MDL54/T41

□ BFP57/T41

■ MDL53/T41

□ CHECK

57

*FIGURE 7*

**European Patent Office**

## EUROPEAN SEARCH REPORT

Application Number

EP 07 29 0060

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FRISH M, BOHN M, MELCHINGER AE: "PLABSIM: Software for simulation of Marker-Assisted Backcrossing" JOURNAL OF HEREDITY, vol. 91, 2000, pages 86-87, XP007902079 | 1,3,5, 7-34 | INV. G06F19/00 A01H1/04 |
| Y | * page 86, middle column, line 8 - page 87, middle column, line 34 * ----- | 2,4,6 | |
| X | SERVIN B, DILLMANN C, DECOUX G, HOSPITAL F: "MDM: A program to compute fully informative genotype frequencies in complex breeding schemes" JOURNAL OF HEREDITY, vol. 93, no. 3, 2002, pages 227-228, XP007902080 | 1,3,5, 7-34 | |
| Y | * page 227, middle column, line 25 - right-hand column, line 53 * ----- | 2,4,6 | |
| Y | US 5 492 547 A (JOHNSON RICHARD [US]) 20 February 1996 (1996-02-20) * column 5, line 20 - line 43 * * column 6, line 25 - line 60 * * column 9, line 1 - column 10, line 14 * * column 15, line 29 - column 17, line 39 * * column 18, line 46 - column 19, line 18 * ----- -/-- | 2,4,6-34 | **TECHNICAL FIELDS SEARCHED (IPC)** G06F A01H C12Q |

The present search report has been drawn up for all claims

| Place of search | Date of completion of the search | Examiner |
|---|---|---|
| Munich | 19 September 2007 | Kürten, Ivayla |

EPO FORM 1503 03.82 (P04C01)

**European Patent Office**

## EUROPEAN SEARCH REPORT

Application Number

EP 07 29 0060

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2005/144664 A1 (SMITH OSCAR S [US] ET AL) 30 June 2005 (2005-06-30) * page 1, paragraph 9 - page 2, paragraph 17 * * page 8, paragraph 88 - page 9, paragraph 92 * * page 10, paragraph 97 - paragraph 101 * * page 19, paragraph 197 - page 20, paragraph 214 * ----- | 2,4,6-34 | |
| A | HOSPITAL F, DILLMANN C, MELCHINGER AE: "A general algorithm to compute multilocus genotype frequencies under various mating schemes." COMPUTER APPLICATIONS IN THE BIOSCIENCES, vol. 12, no. 6, 1996, pages 455-462, XP009082303 * abstract * * page 455, right-hand column, line 33 - page 456, left-hand column, line 30 * ----- | 1-34 | |
| A | FRISCH M, MELCHINGER AE: "Marker-based prediction of the parental genome contribution to inbred lines derived from biparental crosses." GENETICS, vol. 174, October 2006 (2006-10), pages 795-803, XP007902081 * page 796, left-hand column, line 1 - page 797, right-hand column, line 34 * * page 801, line 4 - line 14 * ----- | 1-34 | TECHNICAL FIELDS SEARCHED (IPC) |
| A | MAURER H P ET AL: "PLABSOFT: SOFTWARE FOR SIMULATION AND DATA ANALYSIS IN PLANT BREEDING" PROCEEDINGS OF THE EUCARPIA GENERAL CONGRESS, X, XX, no. 17TH, 2004, pages 359-362, XP009083615 * the whole document * ----- | 2,4,6-34 | |

The present search report has been drawn up for all claims

| Place of search | Date of completion of the search | Examiner |
|---|---|---|
| Munich | 19 September 2007 | Kürten, Ivayla |

European Patent
Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**European Patent Office**

**LACK OF UNITY OF INVENTION SHEET B**

Application Number

EP 07 29 0060

The Search Division considers that the present European patent application does not comply with the requirements of unity of invention and relates to several inventions or groups of inventions, namely:

```
1. claims: 1,3,5,7-34

        A method of calculating a probability or a frequency
        distribution of the potential progeny genotype of a breeding
        pair.
                        ---

2. claims: 2,4,6-34

        A method of calculating a genetic value distribution of the
        potential progeny genotype of a breeding pair.
                        ---
```

## ANNEX TO THE EUROPEAN SEARCH REPORT
## ON EUROPEAN PATENT APPLICATION NO.

EP 07 29 0060

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2007

| Patent document cited in search report | | Publication date | Patent family member(s) | Publication date |
|---|---|---|---|---|
| US 5492547 | A | 20-02-1996 | NONE | |
| US 2005144664 | A1 | 30-06-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6399855 B, Beavis
- US 5385835 A
- US 5492547 A
- US 5981832 A
- US 20030005479 A
- US 7135615 B

**Non-patent literature cited in the description**

- **BEAVIS.** Proc. 49th Ann Corn and Sorghum Res Conf. American Seed Trade Association, 1994, 250-266
- **EDWARDS et al.** *Genetics,* 1987, vol. 115, 113-125
- **FISCH et al.** *Genetics,* 1996, vol. 143, 571 577
- **JACCOUD et al.** *Nucleic Acids Res,* 2001, vol. 29, e25
- **LANDER ; SCHORK.** *Science,* 1994, vol. 265, 2037-2048
- Biometrics in plant breeding: applications of molecular markers. **STAM.** Proc. 9th Meeting Eucarpia Section Biometrics. Plant Research International. 1994
- **WAN et al.** *Theoretical and Applied Genetics,* 1989, vol. 77, 889-892